# EUROPEAN PATENT APPLICATION

(11) **EP 4 831 192 A1**
(43) Date of publication of application: **28.10.2026**
(21) Application number: 25736397.8
(22) Date of filing: 03.01.2025
(51) Int. Cl.: C07K 14/18, A61K 39/12, A61P 31/14, G01N 33/68, G01N 33/569

(54) **RECOMBINANT ANTIGEN AGAINST ZIKA/DENGUE VIRUS, AND VACCINE COMPOSITION THEREOF AND USE THEREOF**

(30) Priority: 05.01.2024 CN 202410024322
(71) Applicant: Beijing Benewill Technology Development Co., Ltd., Beijing 100176 (CN)
(72) Inventor: YAN, Jinghua, Beijing 102206 (CN); HUANG, Qingrui, Beijing 102206 (CN)
(74) Representative: Murgitroyd & Company
(86) International application number: PCT/CN2025/070536
(87) International publication number: WO 2025/146159

(57) **Abstract**

A recombinant antigen against Zika/Dengue virus, and a vaccine composition thereof and the use thereof. The recombinant antigen is obtained by means of introducing a specific mutation into the FL fusion loop region and optionally the non-FL fusion loop region of the E protein of a wild-type Zika/Dengue virus. By means of introducing the specific mutation, the FL epitope of the recombinant antigen is disrupted, which can avoid inducing the generation of antibodies against the FL epitope; thus, the purpose of reducing or eliminating the ADE effect is achieved. In addition, the recombinant antigen has a correct E protein conformation, has a protein expression level similar to or higher than that of a wild-type, and can induce a relatively high titer of specific binding antibodies and neutralizing antibodies in an animal body, so that an effective immune protection can be provided, and good clinical application values and industrialization prospects are achieved.

## Description

### Cross-Reference to Related Applications

The pesent application claims priority to Chinese patent application No. 202410024322.3 filed on January 05, 2024 and entitled "RECOMBINANT ANTIGEN AGAINST ZIKA/DENGUE VIRUS, AND VACCINE COMPOSITION THEREOF AND USE THEREOF", which is incorporated by reference herein in its entirety.

### Technical Field

The present application relates to the field of biotechnology, and in particular, to a recombinant antigen against Zika/Dengue virus, a vaccine composition thereof, and use thereof.

### Background Art

Zika virus (ZIKV) is a mosquito-borne virus belonging to the *Flavivirus* genus of the *Flaviviridae* family, and was first discovered in the Zika forest of Uganda, Africa. In 2007, ZIKV was only sporadically endemic in tropical regions of Africa and Asia, until an outbreak occurred in French Polynesia in 2013, followed by an outbreak in the Americas during 2015-2016, which spread to 84 countries worldwide, including China. However, to date, no vaccine or therapeutic drug is available. Although the global incidence of ZIKV infection has declined, ZIKV continues to pose a threat to people living in endemic regions. Therefore, there is an urgent need for the development of ZIKV vaccines.

Dengue virus (DV) is the causative agent of Dengue fever (DF), which is transmitted primarily by vector insects such as *Aedes aegypti* and *Aedes albopictus*, and is widely distributed in tropical and subtropical regions. In some countries, DV infection has even become a leading cause of hospitalization and death in children. According to the World Health Organization (WHO), Dengue is currently endemic in more than 100 countries and regions, with 2.5-3 billion people living in DF-endemic areas worldwide. Approximately 390 million people are infected per year, with 100 million developing clinical symptoms, over 2 million progressing to severe DF, and more than 20,000 dying. ZIKV and DV have similar structures, both being spherical, enveloped, with an envelope (E) protein on the envelope surface. The internal viral genome of both is a single-stranded positive-sense RNA of 11 kb in length, containing a single open reading frame. The translated polyprotein can be cleaved into three structural proteins (C, prM, and E) and seven non-structural proteins (NS1, NS2A, NS2B, NS3, NS4A, NS4B, and NS5). Among these, the C protein forms the nucleocapsid. The prM protein is cleaved during virus maturation to generate the M protein, which remains anchored in the inner layer of the viral envelope. PrM serves as an important co-factor for inducing protective immunity and also facilitates proper folding and structural stabilization of the E protein. The E protein is the major glycoprotein of the viral envelope, associated with viral cell tropism, hemagglutination, and induction of hemagglutination-inhibiting (HI) antibodies and neutralizing antibodies, and is the primary protective antigen of Zika and Dengue viruses.

The E protein exists as a homodimer, with each monomer comprising three domains, DI, DII, and DIII, respectively, wherein the tip of DII (residues 98-109) contains a highly conserved fusion loop (FL) region, wherein the sequence of the FL region of ZIKV is completely identical to that of DV, both being D98-R99-G100-W101-G102-N103-G104-C105-G106-L107-F108-G109. The FL region plays a critical role in the membrane fusion process during viral entry. During infection, immune cells produce a large number of FL-targeting antibodies.

Dengue virus (DV) belongs to the *Flavivirus* genus of the *Flaviviridae* family, and is classified into four serotypes 1, 2, 3, and 4) based on antigenic differences. DV infection exhibits a classic phenomenon known as antibody-dependent infection enhancement (ADE). When infected with a serotype of virus, e.g., DV-1, the immune cells in the body produce anti-DV-1 antibodies. However, upon subsequent infection with a different serotype, e.g., DV-2, these pre-existing anti-DV-1 antibodies not only fail to neutralize DV-2 but also exacerbate the infection.

Currently, Dengue vaccines include Dengvaxia from Sanofi Pasteur and DVax Takeda Pharmaceutical Company, which has just been approved this year. Both are live attenuated vaccines sharing a similar development strategy, with the difference that Dengvaxia uses a yellow fever attenuated strain 17D as the vaccine viral backbone, while DVax uses a DV-2 attenuated strain PDK-53 as the vaccine viral backbone. DVax enhances the immune response and protection against DV-2, but provides weaker protection against DV-3 and DV-4. Dengvaxia has exhibited significant ADE safety concerns in individuals without prior Dengue infection. More than ten cases of severe Dengue fever or even death occurred in children in the Philippines after vaccination with Dengvaxia. Therefore, this vaccine is strictly restricted to individuals with a history of Dengue infection. In the newly published 5-year follow-up data after vaccination, the DVax vaccine also showed potential ADE safety risks in individuals without prior Dengue infection.

Numerous studies have shown that the hydrophobic fusion loop (FL) of the E protein is the main epitope responsible for inducing the ADE effect, and is also a major dominant epitope on the E protein. Among the antibodies screened from Dengue-infected individuals, 41% (46/112) antibodies targeted the FL (Dejnirattisai W. et al. Nature Immunology (2016)). To completely eliminate the vaccine ADE effect, the general approach is to mutate the FL epitope so that it cannot induce FL antibodies. At the same time, the mutation must not disrupt the remaining epitopes of the E protein or cause a significant reduction in protein expression, to ensure that the vaccine can induce sufficient neutralizing antibodies and generate immune protection. However, the FL epitope plays a critical role in inducing fusion between the viral envelope and the host cell membrane during flavivirus entry into host cells, and its amino acid sequence D98-R99-G100-W101-G102-N103-G104-C105-G106-L107-F108-G109 is highly conserved across the entire genus *Flavivirus*. In the inventors' previous studies, when W101 was mutated to any of the other 25 amino acids, the E protein was barely expressed. Throughout the genus *Flavivirus*, only a very small number of variant sequences exist in the FL of insect-specific flaviviruses that are distantly related to Dengue/Zika virus. However, when these variant sequences were applied to Dengue vaccines, the mutations resulted in a significant decrease in E protein expression, such that the vaccines failed to provide effective immune protection.

In view of the above problems in the prior art, there is an urgent need for the development of a Zika/Dengue vaccine capable of avoiding the ADE effect while maintaining high immunogenicity.

### Summary of the Invention

### Purpose

In view of the problems or needs in the prior art, the present application aims to provide a recombinant antigen against Zika/Dengue virus that avoids the ADE effect while achieving high expression levels and high immunogenicity, as well as a vaccine composition based on the recombinant antigen and use thereof.

### Solutions

To achieve the above objective, the present application introduces an FL epitope antibody to directionally induce a mutation in the viral FL fusion loop region during Zika virus culture. Since the amino acid sequence of the Zika virus FL epitope is identical to that of the Dengue virus FL epitope, the inventors subsequently introduce site mutations occurring in the Zika virus FL epitope and other positions into Dengue vaccines, thereby obtaining a recombinant E protein antigen against Zika virus/Dengue virus. The obtained recombinant E protein antigen against Zika virus/Dengue virus is expressed efficiently, exhibits high immunogenicity against Zika virus/Dengue virus, and avoids the induction of FL epitope antibody production, thereby achieving the effect of reducing or eliminating the ADE effect.

Specifically, the present application provides the following technical solutions.

In a first aspect, the present application provides a recombinant antigen having a full-length E protein sequence of Zika virus or Dengue virus, wherein the E protein has site mutations selected from the group consisting of:
a G106 site mutation in the fusion loop (FL) region;
W101 and G106 double site mutations in the FL fusion loop region;
W101 and G106 double site mutations in the FL fusion loop region and a mutation at amino acid position 125 in the non-FL fusion loop region;
G102 and G106 double site mutations in the FL fusion loop region;
N103 and G106 double site mutations in the FL fusion loop region;
W101, N103 and G106 triple site mutations in the FL fusion loop region.

In a preferred embodiment, the G106 site mutation in the FL fusion loop region is a G106V mutation;
In a preferred embodiment, the W101 and G106 double site mutations in the FL fusion loop region are selected from the group consisting of: (1) W101R and G106V, (2) W101G and G106V, and (3) W101L and G106V;
In a preferred embodiment, the W101 and G106 double site mutations in the FL fusion loop region and the mutation at amino acid position 125 in the non-FL fusion loop region are: W101R and G106V double site mutations in the FL fusion loop region and a mutation of the amino acid at position 125 in the non-FL fusion loop region to valine;
In a preferred embodiment, the G102 and G106 double site mutations in the FL fusion loop region are: G102R and G106V double site mutations;
In a preferred embodiment, the N103 and G106 double site mutations in the FL fusion loop region are selected from the group consisting of: (1) N103T and G106V, (2) N103H and G106V, (3) N103K and G106V, (4) N103P and G106V, and (5) N103Y and G106V;
In a preferred embodiment, the W101, N103 and G106 triple site mutations in the FL fusion loop region are selected from the group consisting of: (1) W101G, N103T and G106V, and (2) W101G, N103K and G106V.

In some preferred specific embodiments, the recombinant antigen has a full-length E protein sequence of Dengue virus serotype 1, and the E protein has a site mutation selected from the group consisting of:
a G106V mutation;
W101R and G106V double site mutations;
W101G and G106V double site mutations;
W101L and G106V double site mutations;
W101R, G106V and L125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

In other preferred specific embodiments, the recombinant antigen has a full-length E protein sequence of Dengue virus serotype 2, and the E protein has a site mutation selected from the group consisting of:
a G106V mutation;
W101R and G106V double site mutations;
W101G and G106V double site mutations;
W101L and G106V double site mutations;
W101R, G106V and M125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

In other preferred specific embodiments, the recombinant antigen has a full-length E protein sequence of Dengue virus serotype 3, and the E protein has a site mutation selected from the group consisting of:
a G106V mutation;
W101R and G106V double site mutations;
W101G and G106V double site mutations;
W101L and G106V double site mutations;
W101R, G106V and I125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

In other preferred specific embodiments, the recombinant antigen has a full-length E protein sequence of Dengue virus serotype 4, and the E protein has a site mutation selected from the group consisting of:
a G106V mutation;
W101R and G106V double site mutations;
W101G and G106V double site mutations;
W101L and G106V double site mutations;
W101R, G106V and I125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

Further preferably, when the recombinant antigen has the full-length E protein sequence of Zika virus, the recombinant antigen further comprises a full-length or partial prM protein sequence or M protein sequence of Zika virus.

Further preferably, when the recombinant antigen has the full-length E protein sequence of Dengue virus, the recombinant antigen further comprises a full-length or partial prM protein sequence or M protein sequence of the corresponding serotype of Dengue virus.

In a further preferred specific embodiment, when the recombinant antigen has the full-length E protein sequence of Dengue virus serotype 1, the recombinant antigen has the amino acid sequence as set forth in SEQ ID NOs: 2-14;

In a further preferred specific embodiment, when the recombinant antigen has the full-length E protein sequence of Dengue virus serotype 2, the recombinant antigen has the amino acid sequence as set forth in SEQ ID NOs: 16-28;

In a further preferred specific embodiment, when the recombinant antigen has the full-length E protein sequence of Dengue virus serotype 3, the recombinant antigen has the amino acid sequence as set forth in SEQ ID NOs: 30-42;

In a further preferred specific embodiment, when the recombinant antigen has the full-length E protein sequence of Dengue virus serotype 4, the recombinant antigen has the amino acid sequence as set forth in SEQ ID NOs: 44-56.

In a second aspect, the present application provides a method for preparing the recombinant antigen according to the first aspect described above, comprising the following steps:
adding a Kozak sequence and a signal peptide coding sequence at the 5' end, and a histidine tag coding sequence and a stop codon at the 3' end of the nucleotide sequence encoding the recombinant antigen of any one of claims 1-8; performing cloning and expression; screening for correct recombinants; transfecting the recombinants into expression system cells for expression;
collecting the cell culture supernatant; and isolating the recombinant antigen from the cell culture supernatant.

Preferably, the expression system cell is a mammalian cell, an insect cell, a yeast cell, or a bacterial cell;
optionally, the mammalian cell is an HEK293T cell, a 293F series cell, or a CHO cell; further
optionally, the 293F series cell is an HEK293F cell, a Freestyle293F cell, or an Expi293F cell;
optionally, the insect cell is an sf9 cell, an Hi5 cell, an sf21 cell, or an S2 cell;
optionally, the yeast cell is a *Pichia pastoris* cell or a yeast cell modified therefrom;
optionally, the bacterial cell is an *Escherichia coli* cell.

In possible embodiments, the signal peptide can be any signal peptide commonly used in the art; for example, the signal peptide can be a signal peptide of which the amino acid sequence is as set forth in one of SEQ ID NOs: 57-61.

In a third aspect, the present application provides a polynucleotide encoding the recombinant antigen according to the first aspect described above. The polynucleotide is not limited by the method used to produce it; for example, it can be obtained using genetic engineering recombinant techniques or chemical synthesis methods.

The polynucleotide can be DNA or mRNA, preferably mRNA.

In preferred embodiments, the polynucleotide is a DNA having a sequence as set forth in any one of SEQ ID NOs: 62-113, or an mRNA corresponding to the DNA.

Specifically, when the recombinant antigen has an amino acid sequence as set forth in SEQ ID NOs: 2-14, the polynucleotide encoding the recombinant antigen is a DNA having a sequence as set forth in SEQ ID NOs: 62-74, or an mRNA corresponding to the DNA; when the recombinant antigen has an amino acid sequence as set forth in SEQ ID NOs: 16-28, the polynucleotide encoding the recombinant antigen is a DNA having a sequence as set forth in SEQ ID NOs: 75-87, or an mRNA corresponding to the DNA; and/or, when the recombinant antigen has an amino acid sequence as set forth in SEQ ID NOs: 30-42, the polynucleotide encoding the recombinant antigen is a DNA having a sequence as set forth in SEQ ID NOs: 88-100, or an mRNA corresponding to the DNA; and/or, when the recombinant antigen has an amino acid sequence as set forth in SEQ ID NOs: 44-56, the polynucleotide encoding the recombinant antigen is a DNA having a sequence as set forth in SEQ ID NOs: 101-113, or an mRNA corresponding to the DNA.

The "mRNA corresponding to the DNA" described above refers to an mRNA having a sequence formed by replacing all "T" in a designated DNA sequence with "U".

In a fourth aspect, the present application provides an expression cassette, a recombinant vector, a transgenic cell line, a recombinant bacterium, or a recombinant virus comprising the polynucleotide according to the third aspect described above.

In a fifth aspect, the present application provides a vaccine or immunogenic composition, comprising the recombinant antigen according to the first aspect described above, or the polynucleotide according to the third aspect described above, or the expression cassette, the recombinant vector, the transgenic cell line, the recombinant bacterium, or the recombinant virus according to the fourth aspect described above, as an active ingredient.

In specific embodiments, in addition to the active ingredient described above, the vaccine or immunogenic composition further comprises a physiologically acceptable vehicle, an adjuvant, an excipient, a carrier, and/or a diluent.

In some possible embodiments, the vaccine or immunogenic composition is a recombinant protein vaccine, comprising the recombinant antigen according to the first aspect described above and an adjuvant.

Preferably, the adjuvant is one or more selected from the group consisting of: an aluminum adjuvant, an MF59 adjuvant, an MF59-like adjuvant, a CpG adjuvant, a QS-21-containing adjuvant, AS series adjuvants (e.g., AS01 series adjuvants), and nanoparticle adjuvants.

In other possible embodiments, the vaccine or immunogenic composition is a DNA vaccine, comprising:
(i) a eukaryotic expression vector; and
(ii) a DNA sequence encoding the recombinant antigen according to the first aspect described above, constructed into the eukaryotic expression vector.

Preferably, the DNA sequence encoding the recombinant antigen according to the first aspect described above is the DNA sequence as set forth in any one of SEQ ID NOs: 62-113.

Preferably, the eukaryotic expression vector is selected from the group consisting of pGX0001, pVAX1, pCAGGS, and pcDNA series vectors.

In other possible embodiments, the vaccine or immunogenic composition is an mRNA vaccine, comprising:
(I) an mRNA sequence encoding the recombinant antigen according to the first aspect described above; and
(II) a lipid nanoparticle.

Preferably, the mRNA sequence encoding the recombinant antigen according to the first aspect described above is an mRNA sequence corresponding to the DNA sequence as set forth in any one of SEQ ID NOs: 62-113.

In other possible embodiments, the vaccine or immunogenic composition is a viral vector vaccine, comprising:
(1) a viral backbone vector; and
(2) a DNA sequence encoding the recombinant antigen according to the first aspect described above, constructed into the viral backbone vector.

Preferably, the DNA sequence encoding the recombinant antigen according to the first aspect described above is the DNA sequence as set forth in any one of SEQ ID NOs: 62-113.

Preferably, the viral backbone vector is selected from one or more of the following viral vectors: an adenoviral vector, a lentiviral vector, a poxvirus vector, an influenza virus vector, and an adeno-associated viral vector.

In one possible embodiment, the vaccine or immunogenic composition is in the form of a nasal spray, an oral formulation, a suppository, or a parenteral formulation.

Preferably, the nasal spray is selected from the group consisting of an aerosol, a spray, and a dry powder inhaler.

Preferably, the oral formulation is selected from the group consisting of a tablet, a powder, a pill, a granule, a soft/hard capsule, a film coating, and an ointment.

Further preferably, the tablet is a sublingual tablet.

Further preferably, the granule is a fine granule.

Further preferably, the powder is a pulvis.

Further preferably, the pill is a pilule.

Preferably, the parenteral formulation is a transdermal formulation, an ointment, a plaster, a topical liquid, an injectable formulation; further preferably, the injectable formulation is a bolus injectable formulation.

In a sixth aspect, the present application provides use of the recombinant antigen according to the first aspect described above, or the polynucleotide according to the third aspect described above, or the expression cassette, the recombinant vector, the transgenic cell line, the recombinant bacterium, or the recombinant virus according to the fourth aspect described above in the manufacture of a medicament for detecting, preventing and/or treating Zika virus or Dengue virus infection.

Preferably, the medicament is a vaccine.

In a seventh aspect, the present application provides a method for preventing and/or treating Zika virus or Dengue virus infection, the method comprising: administering to a subject in need thereof a prophylactically and/or therapeutically effective amount of the following substances: the recombinant antigen according to the first aspect described above, the polynucleotide according to the third aspect described above, the expression cassette, the recombinant vector, the transgenic cell line, the recombinant bacterium, or the recombinant virus according to the fourth aspect described above, and/or the vaccine or immunogenic composition according to the fifth aspect described above.

The "prophylactically and/or therapeutically effective amount" may vary depending on the subject administered, the subject organ, symptoms, method of administration, etc., and may be determined based on the physician's judgment, taking into account the type of dosage form, method of administration, age and weight, and symptoms of the patient, etc.

### Beneficial effects

The recombinant antigen against Zika/Dengue virus of the present application effectively eliminates the FL epitope in the E protein, avoids inducing the production of FL epitope antibodies that cause ADE, thereby significantly reducing or eliminating the ADE effect. At the same time, the recombinant antigen can maintain the correct conformation of the E protein and has a protein expression level similar to or higher than that of the wild type. As confirmed by animal immunization experiments, the Zika/Dengue vaccine based on the recombinant antigen can induce high specific antibody titers and neutralizing antibody titers in animals, thereby providing effective immune protection, and the cross-reactive antibody titers induced against E proteins of other serotypes are significantly reduced, and thus the ADE risk of the vaccine is also significantly reduced.

Furthermore, the vaccines obtained based on the recombinant antigen provided by the present application can include various forms, such as recombinant protein vaccines, nucleic acid vaccines (e.g., mRNA vaccines), viral vector vaccines (e.g., adenoviral vector vaccines), live attenuated or inactivated vaccines based on the antigen sequence, chimeric vaccines with other backbones, etc. These vaccines can be used for preparing Zika and/or Dengue vaccines with the ADE effect eliminated, exhibiting good clinical application value and industrialization prospects.

### Brief Description of the Drawings

One or more examples are exemplified by the pictures in the accompanying drawings that correspond thereto and are not intended to be limiting of the examples. As used herein, the word "exemplary" means "serving as an example, embodiment, or illustration". Any example described herein as "exemplary" is not necessarily to be construed as preferred or advantageous over other examples.
FIG. 1 shows the antigen epitope detection results of the DV1 mRNA vaccine prepared in Example 1, as detected in Example 4.
FIG. 2 shows the antigen epitope detection results of the DV2 mRNA vaccine prepared in Example 1, as detected in Example 4.
FIG. 3 shows the antigen epitope detection results of the DV3 mRNA vaccine prepared in Example 1, as detected in Example 4.
FIG. 4 shows the antigen epitope detection results of the DV4 mRNA vaccine prepared in Example 1, as detected in Example 4.
FIG. 5 shows the specific antibody titers against the viral E protein induced by each DV mRNA vaccine, as detected by enzyme-linked immunosorbent assay in Example 6.
FIG. 6 shows the neutralizing antibody titers against the corresponding serotype DV induced by each DV mRNA vaccine, as detected by viral micro-neutralization assay in Example 7.
FIG. 7 shows the cross-reactive antibody titers against DV2/3/4 and ZIKV E proteins induced by the DV1 mRNA vaccine prepared in Example 1, as detected in Example 8.
FIG. 8 shows the cross-reactive antibody titers against DV1/3/4 and ZIKV E proteins induced by the DV2 mRNA vaccine prepared in Example 1, as detected in Example 8.
FIG. 9 shows the cross-reactive antibody titers against DV1/2/4 and ZIKV E proteins induced by the DV3 mRNA vaccine prepared in Example 1, as detected in Example 8.
FIG. 10 shows the cross-reactive antibody titers against DV1/2/3 and ZIKV E proteins induced by the DV4 mRNA vaccine prepared in Example 1, as detected in Example 8.
FIG. 11 shows the neutralizing antibody titers against the corresponding serotype of DV induced by DV1-EM3-1 and DV2-EM3 mRNA vaccines, as detected by viral micro-neutralization assay in Example 9.
FIG. 12 shows the cross-reactive antibody titers against DV2/3/4 and ZIKV E proteins induced by the DV1-EM3-1 mRNA vaccine prepared in Example 1, as detected in Example 9.
FIG. 13 shows the cross-reactive antibody titers against DV1/3/4 and ZIKV E proteins induced by the DV2-EM3 mRNA vaccine prepared in Example 1, as detected in Example 9.
FIG. 14 shows the ADE effect at the cellular level against DV2/3/4 viruses induced by the immune sera of the DV1 mRNA vaccine prepared in Example 1, as detected in Example 10.
FIG. 15 shows the ADE effect at the cellular level against DV1/3/4 viruses induced by the immune sera of the DV2 mRNA vaccine prepared in Example 1, as detected in Example 10.
FIG. 16 shows the ADE effect at the cellular level against DV1/2/4 viruses induced by the immune sera of the DV3 mRNA vaccine prepared in Example 1, as detected in Example 10.
FIG. 17 shows the ADE effect at the cellular level against DV1/2/3 viruses induced by the immune sera of the DV4 mRNA vaccine prepared in Example 1, as detected in Example 10.
FIG. 18 shows the ADE effect in vivo against DV2 virus induced by the immune sera of the DV4 mRNA vaccine prepared in Example 1, as detected in Example 11.
FIG. 19 shows the antibody levels against the FL epitope induced by the tetravalent Dengue mRNA vaccine, as detected using mouse sera obtained from Example 12 in Example 14.
FIG. 20 shows the neutralizing antibody titers against four serotypes of DV induced by the tetravalent Dengue mRNA vaccine, as detected using mouse sera obtained from Example 12 in Example 15.
FIG. 21 shows the challenge protection efficacy against DV1 and DV2 viruses induced by the tetravalent Dengue mRNA vaccine described in Example 12, as detected in Example 16.
FIG. 22 shows the antigen epitope and antigen expression detection results of the DV1 mRNA vaccine prepared in Example 17, as detected in Example 18.
FIG. 23 shows the antigen epitope and antigen expression detection results of the DV2 mRNA vaccine prepared in Example 17, as detected in Example 18.
FIG. 24 shows the antigen epitope and antigen expression detection results of the DV3 mRNA vaccine prepared in Example 17, as detected in Example 18.
FIG. 25 shows the antigen epitope and antigen expression detection results of the DV4 mRNA vaccine prepared in Example 17, as detected in Example 18.
FIG. 26 shows the challenge protection efficacy against DV2 virus induced by the DV2 mRNA vaccine prepared in Example 17, as detected in Example 19.
FIG. 27 shows the SDS-PAGE results of the Dengue virus-like particles prepared in Example 20, as detected in Example 20.
FIG. 28 shows the negative staining electron microscopy results of the DV1-EM3-1 virus-like particles prepared in Example 20, as detected in Example 21.
FIG. 29 shows the negative staining electron microscopy results of the DV2-EM3-1 virus-like particles prepared in Example 20, as detected in Example 21.
FIG. 30 shows the negative staining electron microscopy results of the DV3-EM3 virus-like particles prepared in Example 20, as detected in Example 21.
FIG. 31 shows the negative staining electron microscopy results of the DV4-EM3 virus-like particles prepared in Example 20, as detected in Example 21.
FIG. 32 shows the neutralizing antibody titers against the corresponding serotype of DV induced by each DV recombinant protein vaccine, as detected by viral micro-neutralization assay in Example 22.
FIG. 33 shows the neutralizing antibody titers against four serotypes of DV induced by the tetravalent DV recombinant protein vaccine, as detected by viral micro-neutralization assay in Example 23.

### Detailed Description of the Invention

In order to make the purpose, technical solutions and advantages of the pesent application clearer, the technical solutions in the embodiments of the pesent application will be described clearly and completely. Obviously, the described embodiments are some of the embodiments of the pesent application, but not all of them. Based on the embodiments of the pesent application, all other embodiments obtained by those skill in the art without creative work are within the scope of the pesent application.

In addition, in order to better explain the pesent application, a lot of specific details are given in the following embodiments. It will be understood by those skilled in the art that the pesent application may be practiced without certain specific details. In some embodiments, materials, elements, methods, means, etc., well known to those skilled in the art, are not described in detail so as to highlight the spirit of the pesent application.

Unless otherwise indicated, the practice of the present application will employ conventional techniques of molecular biology (including recombinant techniques), microbiology, cell biology, biochemistry, and immunology, which are within the skill of the art.

Furthermore, unless otherwise expressly defined elsewhere herein, all technical and scientific terms used herein have the same meaning as commonly understood by one of ordinary skill in the art to which the present application belongs. In order to facilitate easier understanding of the present application, certain technical and scientific terms are specifically defined as follows.

The term "comprise" or variations thereof such as "comprises" or "comprising" shall be understood to include the stated elements or components, without excluding other elements or other components.

The term "about" when used in conjunction with a numerical value means to encompass numerical values within a range having a lower limit that is 5% less than the specified numerical value and an upper limit that is 5% greater than the specified numerical value, including but not limited to ±5%, ±2%, ±1%, and ±0.1%, as such variations are suitable for performing the disclosed methods. The term "and/or" should be understood to mean any one of the alternatives or a combination of any two or more of the alternatives.

As used herein, the term "or" should be understood to have the same meaning as "and/or" as defined above. For example, when separating items in a list, "or" or "and/or" shall be interpreted as inclusive, i.e., including at least one of a number or a list of elements, but also including more than one, and optionally, additional unlisted items.

### Example 1: Preparation of Dengue Virus mRNA Vaccines

In this example, a series of mRNA constructs encoding the full-length Dengue virus prME protein (i.e., comprising prM protein + E protein) were designed, including:
DV1-WT (i.e., wild-type DV1), encoding the prME antigen protein as set forth in SEQ ID NO:1;
DV1-EM3, encoding the prME antigen protein as set forth in SEQ ID NO:3;
DV1-EM3-1, encoding the prME antigen protein as set forth in SEQ ID NO:4;
DV2-WT (i.e., wild-type DV2), encoding the prME antigen protein as set forth in SEQ ID NO:15;
DV2-EM3, encoding the prME antigen protein as set forth in SEQ ID NO:17;
DV2-EM3-1, encoding the prME antigen protein as set forth in SEQ ID NO:18;
DV3-WT (i.e., wild-type DV3), encoding the prME antigen protein as set forth in SEQ ID NO:29;
DV3-EM3, encoding the prME antigen protein as set forth in SEQ ID NO:31;
DV3-EM3-1, encoding the prME antigen protein as set forth in SEQ ID NO:32;
DV4-WT (i.e., wild-type DV4), encoding the prME antigen protein as set forth in SEQ ID NO:43;
DV4-EM3, encoding the prME antigen protein as set forth in SEQ ID NO:45;
DV4-EM3-1, encoding the prME antigen protein as set forth in SEQ ID NO:46.

Next, according to mammalian cell codon bias, the nucleic acid sequences encoding the above recombinant Dengue virus prME antigen proteins were optimized to obtain optimized nucleic acid coding sequences, wherein the optimized nucleic acid coding sequences of DV1-WT, DV2-WT, DV3-WT, and DV4-WT were as set forth in SEQ ID Nos: 114-117, respectively, and the optimized nucleic acid coding sequences of DV1-EM3, DV1-EM3-1, DV2-EM3, DV2-EM3-1, DV3-EM3, DV3-EM3-1, DV4-EM3, and DV4-EM3-1 were as set forth in SEQ ID Nos: 63, 64, 76, 77, 89, 90, 102, and 103, respectively. Then, a Kozak sequence and a signal peptide nucleic acid coding sequence (as set forth in SEQ ID NO:118, which encoding the signal peptide of which the amino acid sequence was as set forth in SEQ ID NO: 58) were added at the 5' end of each nucleic acid coding sequence, and a stop codon was added at the 3' end of each, then gene synthesis was commissioned to GenScript Biotech Corporation. The synthesized gene fragments were recombined into the pHRNT vector (the Invention Patent No. ZL202110224383.0) preserved in the laboratory to obtain template plasmids for preparing mRNA vaccines.

The specific preparation process of the mRNA vaccine refers to the method disclosed in the Invention Patent No. ZL202110224383.0. Briefly, the above template plasmids were linearized by enzymatic digestion, followed by in vitro transcription of mRNA using T7 transcriptase. During the transcription process, a modified mRNA was obtained by substituting uracil with pseudouridine or N1-methylpseudouridine to increase the protein expression of mRNA in vivo. Subsequently, the mRNA was encapsulated into nanoparticles by microfluidic technology. During encapsulation, the aqueous phase was an mRNA solution (50 mM sodium acetate buffer, pH 4.0), and the ethanol phase was a lipid mixture (prepared by a protonatable phospholipid, distearoylphosphatidylcholine, cholesterol, and a PEG-modified phospholipid at a molar ratio of 50:10:38.5:1.5), with the total flow rate of the aqueous phase and the ethanol phase being 12 mL/min, and the volume ratio of the aqueous phase to the ethanol phase being 3:1. Finally, the buffer of the encapsulated mRNA vaccine was exchanged to PBS using a dialysis bag. The encapsulated and free mRNA concentrations were determined using Thermo Fisher's Quan-iT RiboGreen RNA Assay Kit, and then the mRNA encapsulation efficiency was calculated. The results showed that the encapsulation efficiency met the standards for mRNA vaccines. Thereafter, the encapsulated mRNA was stored at 4 °C for later use.

### Example 2: Expression, Isolation, and Purification of Detection Antibodies

In this example, the heavy chain and light chain expression plasmids of detection antibodies Z5 (Wang, Q. H. et al. Sci Transl Med 8, (2016)), Z6 (Dai, L. et al. Nat Immunol 22, 958-968, (2021)), 4G2 (Dai, L. P. et al. Nat Immunol 22, 958-968, (2021)), MZ24 (Dussupt, V. et al. Nat Med 26, 228-35, (2020)), Ab513 (Robinson, L. N. et al. Cell 162, 493-504, (2015)), 1F4 (Fibriansah, G. et al. Embo Mol Med 6, 358-371, (2014)), 3H5 (Renner, M. et al. Nature Immunology 19, 1248-56, (2018)), MZ-1 (Dussupt, V. et al. Nat Med 26, 228-35, (2020)), MZ-4 (Dussupt, V. et al. Nat Med 26, 228-35, (2020)), 5J7 (Fibriansah, G. et al. Embo Journal 31, 767-779, (2012)), and 5H2 (Cockburn, J. J. B. et al. Embo Journal 31, 767-779, (2012)) targeting different epitopes of the Dengue virus prME protein were respectively constructed according to the antibody heavy chain and light chain sequences and the method for constructing their expression plasmids disclosed in the literatures.

Among these, Z5, Z6, and 4G2 antibodies recognize the FL epitope of Zika and Dengue viruses, while the remaining antibodies (MZ-1, MZ-4, MZ24, Ab513, 1F4, 3H5, 5J7, and 5H2) are all potently neutralizing antibodies that recognize non-FL epitopes.

### Antibody expression

14-16 h before transfection, the 293T cells with a relatively high density were sub-plated (for example, the 293T cells at 100% confluence in a 10-cm culture dish were passaged at a ratio of 1:3). After 14-16 h, when the cell density reached more than 70%, transfection was performed. During transfection, the heavy chain and light chain expression plasmids of the antibodies constructed as described above were co-transfected into 293T cells at a ratio of 2:3. 4-6 hours after transfection, the cells were washed twice with PBS, and the medium was changed to serum-free DMEM medium for continued culture. Cell supernatants were collected on day 3 and day 7 after transfection, respectively, and then centrifuged to remove cell debris. The antibody supernatants obtained from the above two collections were mixed for subsequent purification of antibody proteins.

### Antibody purification

A Protein A (5 mL) HP affinity column (purchased from GE Company) was connected to an AKTA Purifier/Explorer/FPLC/START (GE Company). The operation procedure on the instrument was as follows: the 20% ethanol in the column was flushed out with water, and then the column was equilibrated with 20 mM Na₃PO₄ buffer at pH 7.0. Once the conductivity on the instrument had stabilized, the above antibody supernatant was then injected via a 10 mL sample loop to bind to Protein A at a flow rate of 2 mL/min. After the UV baseline had stabilized, approximately 0.8 mL of 1M Tris pH 9.0 buffer was added to the subsequent collection tube (a collection volume of approximately 3.2 mL). The program was then set to use 100% 0.1 M Gly at pH 3.0 to elute the antibodies bound to the column. The resulting eluent was collected. Subsequently, the antibody buffer was exchanged into PBS by concentration and buffer exchange. The resulting antibody solution could be used directly or aliquoted and stored in a -80 °C freezer for later use.

### Example 3: Expression and Purification of DV and ZIKV E Proteins

In this example, the E protein expression plasmids of Dengue and Zika viruses were constructed according to the method for soluble expression of DV and ZIKV E protein dimers disclosed in the literature (Kudlacek, S. T. et al. Sci Adv 7, (2021)).

Specifically, according to mammalian cell codon bias, the genes encoding amino acids 1-395 of the E protein of each of DV1 (strain THSTI-TRC-DV1-08, GenBank: OP310803.1), DV2 (strain New Guinea C, GenBank: KM204118.1), DV3 (strain YN02, GenBank: KF824903), and DV4 (strain China Guangzhou B5, GenBank: AF289029), and the gene encoding amino acids 1-404 of the E protein of ZIKV-SMGC-1 (GenBank: KX266255) (wherein, to enable the E protein to form stable dimers, the DV1, DV2, and DV4 E proteins contain an A259C mutation, the DV3 E protein contains an A257C mutation, and the ZIKV E protein contains an A264C mutation) were optimized to obtain the optimized nucleic acid coding sequences of the E protein fragments of each virus, as set forth in SEQ ID NOs:120-124, respectively. Then, a Kozak sequence and a signal peptide coding sequence (as set forth in SEQ ID NO:119, encoding the signal peptide having an amino acid sequence as set forth in SEQ ID NO:60) were added at the 5' end of each of the above nucleic acid coding sequences, and a His tag was added at the 3' end of each for protein purification. Gene synthesis was commissioned to GenScript Biotech Corporation. The synthesized gene fragments were cloned into the pCAGGS vector by double digestion with 5'EcoRI and 3'XhoI to construct the E protein expression plasmids pCAGGS-DV1-sE, pCAGGS-DV2-sE, pCAGGS-DV3-sE, pCAGGS-DV4-sE, and pCAGGS-ZV-sE. Protein expression was performed using HEK293F cells, and protein purification was performed using HisTrap^{™} excel affinity chromatography column and Superdex200 Increase 10/300GL gel filtration chromatography column.

HEK293F cells in healthy condition were selected, seeded into fresh SMM293-TII medium at a density of 1-1.5×10⁶ cells/mL one day prior to transfection, and cultured in a constant-temperature shaker at 37°C, 5% CO₂, 150 rpm. On the day of transfection, the cell density was adjusted to 2.5 ×10⁶ cells/mL. To prepare the transfection reagent (using 10 mL of cells as an example), 10 µg of plasmid was diluted with 150 mM NaCl to a total volume of 250 µL and gently mixed. Sinofection transfection reagent was diluted with 150 mM NaCl to a total volume of 250 µL and gently mixed. The diluted plasmid and the transfection reagent were each allowed to stand separately for about 5 min, then gently mixed to obtain a total volume of 500 µL, followed by standing at room temperature for another 10 min. The transfection mixture was added dropwise to the cell culture medium while gently shaking the culture flask, and after mixing, the flask was returned to the shaker for continued culture. At 24 h after transfection, 350 µL of SMS-293-SUPI feed solution was added, and thereafter, the feed solution (350 µL) was added every 48 h. Five days after transfection, the supernatant was collected for protein purification.

The cell supernatant collected five days after transfection was centrifuged at 8000 rpm, 4 °C for 1 h, and then filtered through a 0.22 µm filter membrane. His affinity chromatography purification: the HisTrap^{™} excel affinity chromatography column was first rinsed with about 3-5 column volumes of water filtered through a 0.22 µm filter membrane. Thereafter, the column was rinsed with HisTrap affinity chromatography equilibration buffer until both the UV and conductivity detection baselines had stabilized, indicating that the column had been equilibrated. Then, the cell supernatant was passed through the column at a flow rate of 1-2 mL/min to allow the protein to bind to the column. After all the supernatant had passed through the column, the column was rinsed with 5-10 column volumes of affinity chromatography equilibration buffer until the UV detection baseline had stabilized. Finally, the column was rinsed sequentially with elution buffers containing 50 mM, 300 mM, and 1 M imidazole. The collected protein fractions were sampled and identified by SDS-PAGE. Further purification was performed using Superdex 200 Increase 10/300 GL gel filtration chromatography. The sample obtained from the affinity chromatography was concentrated to below 800 µL using a 10 kDa ultrafiltration concentrator and then transferred to a 1.5 mL centrifuge tube. After centrifugation at 12,000 g, 4°C for 20 min, the sample was transferred to a new 1.5 mL centrifuge tube, ensuring that no precipitate was collected. Centrifugation was performed again at 12,000 g, 4 °C for 20 min to remove air bubbles in the sample, so that the sample was ready for loading. The Superdex 200 Increase 10/300 GL gel filtration chromatography column was equilibrated with gel filtration chromatography equilibration buffer at a maximum flow rate of 0.5 mL/min until the UV detection baseline had stabilized, and the sample was loaded using a 1 mL loop. After loading, the column was rinsed with 20/50 equilibration buffer (20 mM Tris, 50 mM NaCl) at a flow rate of 0.5 mL/min. Peak collection was performed to collect the sample at the peak position. The collected sample was prepared and identified by SDS-PAGE. The sample identified to be correctly expressed was concentrated using a 10 kDa ultrafiltration concentrator, aliquoted, and stored in a -80°C freezer for later use.

### Example 4: Antigen Expression Detection of Dengue Virus mRNA Vaccines

In this example, a series of Dengue Virus prME mRNA vaccines prepared in Example 1 were transfected into 293T cells, and then the FL epitope antibodies and other neutralizing epitope antibodies prepared in Example 2 were used to detect the epitope status and protein expression level in each prME.

Specifically, HEK293T cells were cultured in DMEM medium containing 10% FBS until the cell density reached >70% confluence, and then transfected with the mRNA vaccine constructed in Example 1. 12 hours after transfection, the cell culture supernatant was discarded, and the cells were digested with trypsin and collected. After washing the cells with PBS, a cell fixation and permeabilization solution (BD Cytofix/Cytoperm^{™} Fixation/Permeabilization Kit) was added and incubated on ice for 20 minutes. After washing the cells twice with 1× Wash Buffer (BD Cytofix/Cytoperm^{™} Fixation/Permeabilization Kit), the antibodies Z5 and Z6 from Example 2, which recognize different epitopes of the DV prME protein, were added and incubated on ice in the dark for 30 minutes. After washing the cells twice with 1× Wash Buffer, FITC-labeled goat anti-human secondary antibody (Abcam, USA) was added and incubated on ice in the dark for 30 minutes. After washing the cells twice with 1× Wash Buffer, the cells were resuspended in PBS, and the cellular fluorescence was detected on a BD FACSAria III flow cytometer. The data were analyzed using FlowJo 7.6.1.

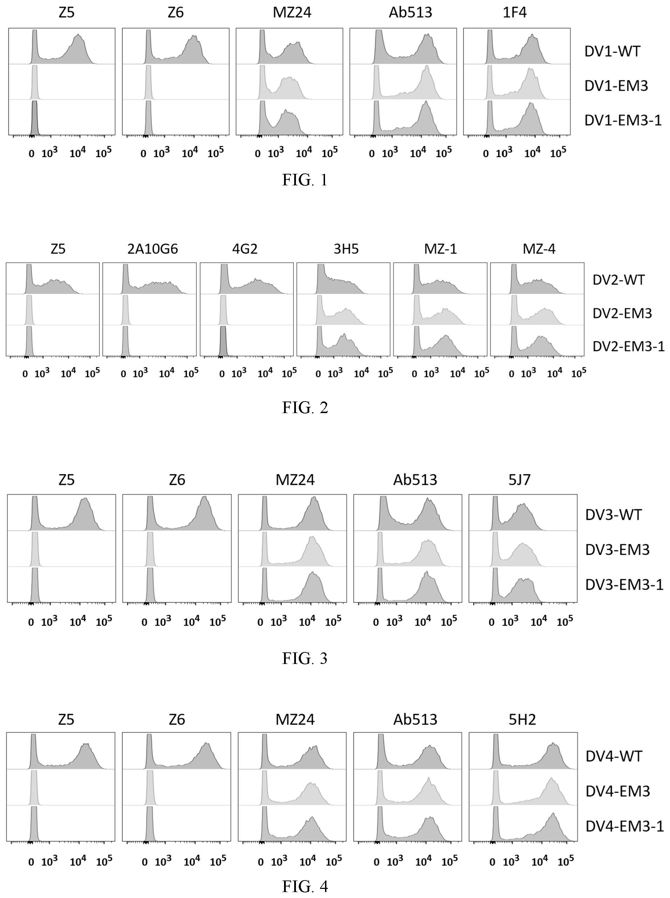

The results were shown in FIGS. 1-4, which showed that:
For the DV1 mRNA vaccine, the FL epitope antibodies (Z5 and Z6) could bind to DV1-WT but not to the two mutants (DV1-EM3 and DV1-EM3-1), indicating that in the two mutants, the FL epitope had been disrupted; other neutralizing epitope antibodies (MZ24, Ab513, and 1F4) could bind to both DV1-WT and the two mutants, indicating that in the two mutants, other neutralizing epitopes remained unchanged, and the protein expression level of each of the two mutants was similar to that of the wild type.

For the DV2 mRNA vaccine, the FL epitope antibodies (Z5, Z6, and 4G2) could bind to DV2-WT but not to the two mutants DV2-EM3 and DV2-EM3-1, indicating that in the two mutants, the FL epitope had been disrupted; other neutralizing epitope antibodies (3H5, MZ-1, and MZ-4) could bind to both DV2-WT and the two mutants, indicating that in the two mutants, other neutralizing epitopes remained unchanged, and the protein expression level of each of the two mutants was similar to that of the wild type.

For the DV3 mRNA vaccine, the FL epitope antibodies (Z5 and Z6) could bind to DV3-WT but not to the two mutants DV3-EM3 and DV3-EM3-1, indicating that in the two mutants, the FL epitope had been disrupted; other neutralizing epitope antibodies (MZ24, Ab513, and 5J7) could bind to both DV3-WT and the two mutants, indicating that in the two mutants, other neutralizing epitopes remained unchanged, and the protein expression level of each of the two mutants was similar to that of the wild type.

For the DV4 mRNA vaccine, the FL epitope antibodies (Z5 and Z6) could bind to DV4-WT but not to the two mutants DV4-EM3 and DV4-EM3-1, indicating that in the two mutants, the FL epitope had been disrupted; other neutralizing epitope antibodies (MZ24, Ab513, and 5H2) could bind to both DV4-WT and the two mutants, indicating that in the two mutants, other neutralizing epitopes remained unchanged, and the protein expression level of each of the two mutants was similar to that of the wild type.

The above results indicated that in all of the above mutants, the FL epitope was disrupted, while other neutralizing antibody epitopes remained unchanged; moreover, the protein expression levels of the mutants were comparable to or higher than those of the wild types. That is, the FL epitope in the recombinant antigen of the present application was disrupted, which avoided inducing the production of FL epitope antibodies that cause ADE, thereby significantly reducing or eliminating the ADE effect. At the same time, the recombinant antigen could normally bind to other neutralizing epitope antibodies, indicating that it had the correct conformation of the E protein. Moreover, the recombinant antigen had a protein expression level similar to or higher than that of the wild type.

### Example 5: Mouse Immunization Experiment with Dengue Virus mRNA Vaccines

In this example, female BALB/c mice aged 6-8 weeks (purchased from Beijing Vital River Laboratory Animal Technology Co., Ltd.) were used for the vaccine immunization experiment. The experiment was divided into an mRNA vaccine immunization group and a negative control group (i.e., Sham group), with 6 mice per group. For each immunization group, a placebo (saline, as Sham group) or the DV mRNA vaccine prepared in Example 1 was administered by intramuscular injection on day 0 and day 14, respectively, with each injection dose of 12 µg per mouse. Blood was collected at week 4 after immunization, and the serum was separated at 4 °C, inactivated at 56 °C for 30 minutes, and stored at -80 °C for later use.

### Example 6: Detection of Specific Antibody Titers in Immunized Mouse Sera by Enzyme-Linked Immunosorbent Assay (ELISA)

In this example, the specific antibody titers against the antigen E protein in sera of immunized mice obtained from Example 5 were detected by ELISA.

Specifically, the following procedure was applied:
(1) the DV or ZIKV E protein prepared in Example 3 was diluted to 2 µg/mL with ELISA coating buffer (Solarbio, C1050) and then added to a 96-well ELISA plate (Corning, 3590) with 100 µL per well; the plate was then placed at 4 °C for 12 hours;
(2) the coating buffer was discarded, and the plate was washed once with PBS; subsequently, a 5% skim milk solution prepared with PBS was added to the 96-well plate with 100 µL per well as a blocking solution, and the plate was then placed at room temperature for 1 hour for blocking; after blocking, the plate was washed once with PBS;
(3) during the blocking in step (2), the mouse serum samples were serially diluted 2-fold with a blocking solution starting from a 10-fold dilution; then 100 µL of the immune serum dilution was added to each well of the ELISA plate, while a blocking solution was added to the negative control group; the plate was incubated at 37°C for 2 hours, and then washed 4 times with PBST;
(4) an HRP-conjugated goat anti-mouse secondary antibody (Abcam, ab6789) diluted 1:2000 with a blocking solution was added and incubated at 37 °C for 1 hour, followed by washing 5-6 times with PBST; subsequently, TMB color development solution was added for color development; after an appropriate reaction time, 2 M hydrochloric acid was added to stop the reaction, and the OD₄₅₀ value was measured on a microplate reader.

The antibody titer was defined as the highest serum dilution at which the reaction value was greater than 2.1 times the negative control value. When the reaction value at the lowest dilution (detection limit) was still less than 2.1 times the background value, the titer of this sample was defined as half of the lowest dilution, i.e., 1:5.

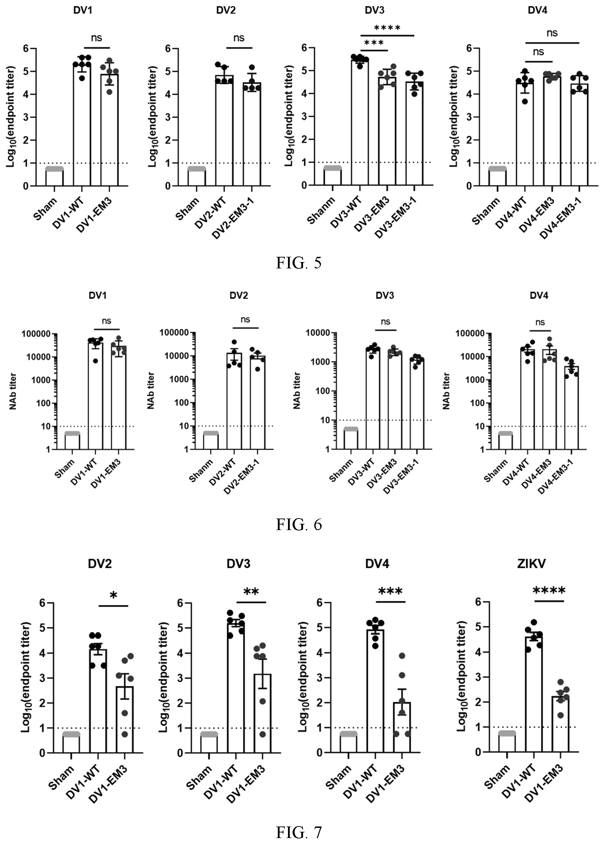

The ELISA results were shown in FIG. 5, which showed that DV1-EM3 induced DV1 E protein-specific antibody titers similar to those of DV1-WT; DV2-EM3-1, DV4-EM3, and DV4-EM3-1 vaccines also induced antibody titers similar to those of the corresponding wild-type vaccines. Compared with DV3-WT, the DV3 E protein-specific antibody titers induced by DV3-EM3 and DV3-EM3-1 were reduced but remained high (>10,000).

The above results indicated that the recombinant antigen of the present application could induce high levels of specific binding antibodies in mice, suggesting that it exhibited high immunogenicity and could induce a high level of immune protective effect.

### Example 7: Detection of Neutralizing Antibody Titers in Immunized Mouse Sera by Dengue Virus Micro-Neutralization Assay

In this example, the neutralizing antibody titers against the corresponding serotypes of Dengue virus in the sera of mice immunized with the mRNA vaccines from Example 5 were quantitatively determined by Dengue virus micro-neutralization assay. The specific operation steps were as follows:
Vero cells were seeded in a 24-well cell culture plate one day in advance, and then used for the neutralization assay on the next day when the cells grew to a confluence of greater than 70%. The serum was serially diluted with DMEM medium (Invitrogen, C11995500BT) containing 1% FBS (Gibco, 10270-106), and the virus was also diluted with the DMEM medium containing 1% FBS. The serum and the virus solution were mixed at an equal volumes of 200 µL each. At the same time, the diluted virus was mixed with an equal volume of blank medium as a positive control. The mixtures were incubated at 37°C for 30 min. After the incubation was complete, the supernatant medium in the Vero cell plate was removed, and the above mixture was added to the cells at 350 µL/well. After incubation in a 37 °C incubator for 1 hour, 150 µL of DMEM medium containing 1% FBS was added to each well, and the plate was placed in a 37° C incubator for continued culture for 3 days. After 3 days, the cell culture plate was taken out, and all the supernatant was discarded, and the cells were washed once with PBS. Then, 150 µL of trypsin was added to each well to digest the cells, and the plate was placed in a constant-temperature incubator at 37 °C for 5 minutes. Then, 150 µL of PBS containing 10% FBS was added to each well to stop the trypsin digestion. The digested cells were pipetted to mix well, and all the cells from the 24-well plate were transferred to a 96-well V-bottom plate, centrifuged, collected, and then washed once with PBS. Then, 100 µL of Fixation and Permeabilization Solution (BD, 554722) was added to each well of a 96-well round-bottom plate. After fixation in the dark at 4 °C for 30 minutes, the cells were collected by centrifugation at 800 g for 5 minutes and washed twice with 1× Perm/Wash Buffer (BD, 554723). AF-488-labeled Z6 antibodies (Z6-AF488) were added at 100 µL per well, and the cells were stained in the dark at 4 °C for 30 minutes. After staining, the cells were collected by centrifugation and washed twice with 1× Perm/Wash Buffer. Finally, the cells were resuspended in 200 µL of PBS, transferred to a flow cytometry tube, and the FITC fluorescence positive rate of the cells was detected using FACS Canto II. Non-linear fitting of the data was performed using GraphPad Prism software to calculate the corresponding serum dilution factor when 50% neutralization of cell infection was achieved, i.e., the neutralizing titer (IC50). When the serum at the lowest dilution still failed to neutralize 50% of cell infection, the IC50 of the sample was defined as half of the lowest dilution.

The results were shown in FIG. 6, which showed that compared with the corresponding WT vaccines, the DV1-EM3, DV2-EM3-1, DV3-EM3, and DV4-EM3 vaccines all induced comparable viral neutralizing antibody titers, while DV3-EM3-1 and DV4-EM3-1 induced slightly lower viral neutralizing antibody titers. In particular, compared with DV3-WT, the DV3-EM3 vaccine induced fewer binding antibodies (FIG. 5), but the neutralizing activity of the vaccine serum did not decrease (FIG. 6), indicating that the DV3-EM3 vaccine induced a higher proportion of potent neutralizing antibodies.

The above results indicated that the recombinant antigen of the present application could induce high levels of neutralizing antibody titers in mice, suggesting that it exhibited high immunogenicity, thereby inducing a high level of immune protective effect.

### Example 8: Determination of Cross-Reactive Antibody Titers

Since the FL epitope is highly conserved in Dengue virus and Zika virus, FL epitope antibodies show broad-spectrum activity, i.e., such antibodies can cross-bind to the E proteins of all serotypes of DV and ZIKV. However, such antibodies generally show poor viral neutralizing activity and readily induce the ADE effect. The FL epitope antibodies induced by any serotype of DV vaccine can cross-bind to the E proteins of the other three serotypes of DV and ZIKV. Therefore, evaluating the cross-reactive antibody titers of a vaccine can reflect the amount of FL epitope antibodies induced by the vaccine and the ADE risk of the vaccine. The cross-reactive antibody titers were determined by ELISA, with the specific method referring to Example 6.

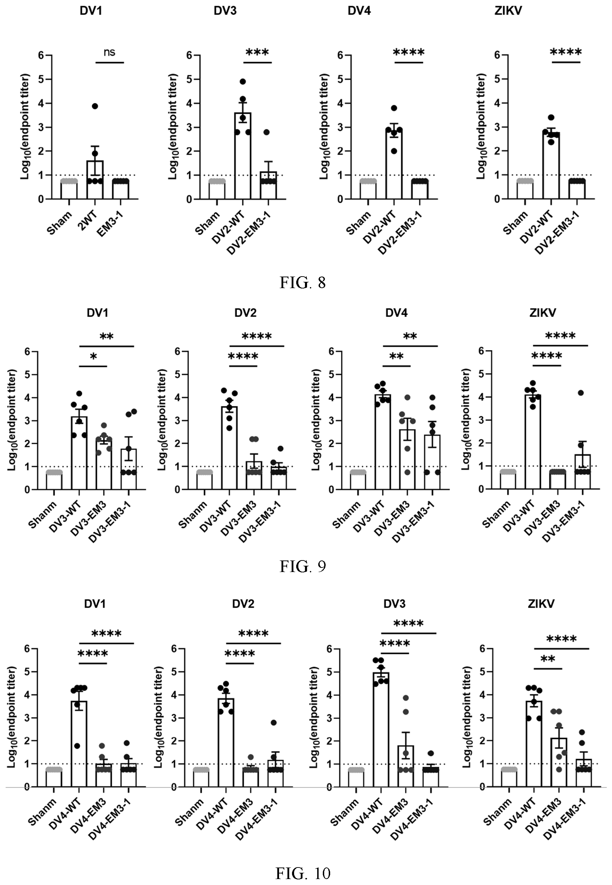

The determination results of the cross-reactive antibody titers induced by immunization with each mRNA vaccine in Example 5 were shown in FIGS. 7-10, which showed that:
for the DV1 vaccine, a large number of antibodies induced by DV1-WT could cross-bind to DV2/3/4 and ZIKV E proteins, whereas the cross-reactive antibody titers of DV1-EM3 were significantly reduced, and cross-binding antibodies were even undetectable in individual mice.

Accordingly, it could be inferred that the ADE risk of the DV1-EM3 vaccine against DV2/3/4 and ZIKV was significantly reduced.

DV2/3/4 vaccines also showed similar results, i.e., compared with the corresponding WT vaccines, the cross-reactive antibody titers induced by each mutant vaccine were significantly and substantially decreased, and the ADE risk thereof was also significantly decreased.

The above results indicated that the cross-reactive antibodies against the E proteins of other serotypes of DV or ZIKV induced by the recombinant antigen of the present application were significantly reduced, thereby substantially reducing the risk of vaccine-induced ADE effect, and thus exhibiting high safety.

### Example 9: Determination of Sera Neutralizing Antibody Titers and Cross-Reactive Antibody Titers of DV1-EM3-1 and DV2-EM3 Vaccine

In this example, the neutralizing antibody titers and cross-reactive antibody titers of the sera of the mice immunized with DV1-EM3-1 and DV2-EM3 vaccines obtained from Example 5 were determined, wherein the method for determining the neutralizing antibody titers referred to Example 7, and the method for determining the cross-reactive antibody titers referred to Example 8.

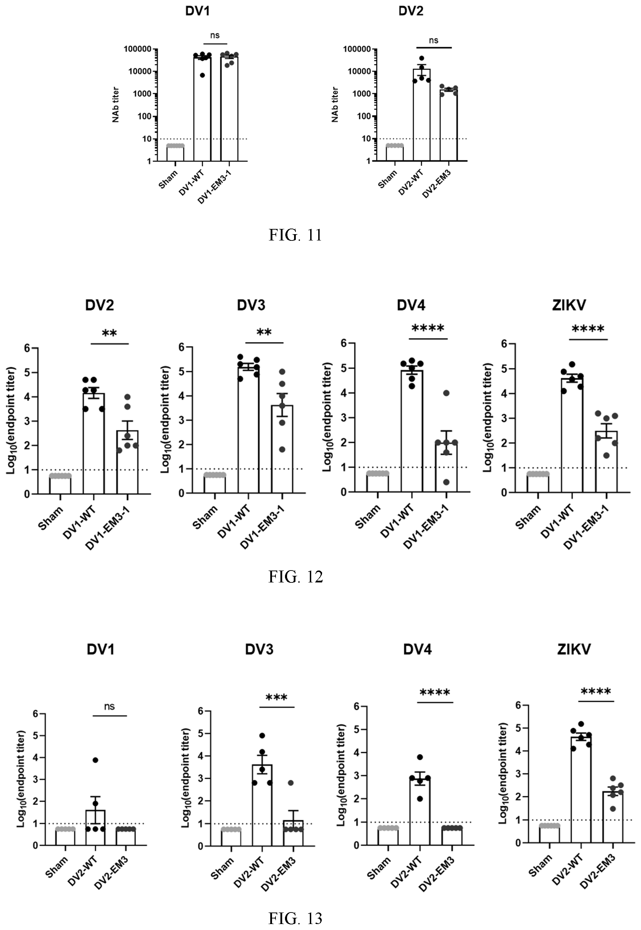

The determination results of serum neutralizing antibody titers were shown in FIG. 11, which showed that compared with the corresponding WT vaccines, DV1-EM3-1 induced comparable viral neutralizing antibody titers, and the neutralizing antibody titers induced by DV2-EM3 were decreased but still remained more than 1,000, both exhibiting good immunogenicity.

The determination results of cross-reactive antibody titers were shown in FIGS. 12-13, which showed that compared with the WT vaccines, the cross-reactive antibody titers against heterologous serotypes of DV and ZIKV induced by DV1-EM3-1 and DV2-EM3 vaccines were significantly and substantially reduced. Accordingly, it could be inferred that the risk of ADE against heterologous serotypes of DV and ZIKV was also substantially reduced.

### Example 10: Evaluation of Vaccine-Induced ADE Effect In Vitro

In this example, female BALB/c mice aged 6-8 weeks (purchased from Beijing Vital River Laboratory Animal Technology Co., Ltd.) were used for the vaccine immunization experiment. The experiment was divided into an mRNA vaccine immunization group and a negative control group (i.e., Sham group), with 8 mice per group. For each immunization group, a placebo (saline, as the Sham group) or the monovalent Dengue mRNA vaccines prepared in Example 1, including DV1-WT, DV2-WT, DV3-WT, DV4-WT, DV1-EM3-1, DV2-EM3-1, DV3-EM3, and DV4-EM3, were administered by intramuscular injection on day 0 and day 21, respectively, with each injection dose of 1 µg per mouse. Blood was collected at week 2 after the booster immunization, and the serum was separated at 4 °C, inactivated at 56 °C for 30 minutes, and stored at -80 °C for later use.

The K562 cell model (ATCC, Cat. No. CCL-243) highly expressing the FcγRIIA receptor was used to determine the ADE effect of the sera from BALB/c mice immunized with the above monovalent mRNA vaccines against the other three serotypes of virus, e.g., the ADE effect of the immune sera of DV1-WT, DV1-EM3, and DV1-EM3-1 against DENV2, 3, and 4 viruses. The specific operation steps were as follows:
The vaccine immune sera were taken out and thawed at 4° C, flicked to mix well, and briefly centrifuged by a centrifuge. In a clean bench, the mouse sera were serially diluted 4-fold in a 96-well U-shape plate (Corning 3799) using RPMI 1640 medium (Invitrogen, C11875500BT) containing 1% fetal bovine serum. DENV virus was diluted to 2×10⁶ FFU/mL with the RPMI 1640 medium containing 1% FBS. The serum dilution was mixed with the three DENV viruses in equal volumes, respectively, and incubated at room temperature for 20 minutes. The cultured K562 cells were centrifuged at 800 g for 5 minutes, resuspended in the RPMI 1640 medium containing 1% FBS, and counted, and adjusted to a density of 4×10⁶ cells/mL. Subsequently, 10 µL of the cell suspension per well was added to the virus-serum mixture, and the plate was incubated in a 37°C cell culture incubator for 1 hour. The RPMI 1640 medium containing 1% FBS was added to 100 µL per well, and the plate was placed in a 37 °C cell culture incubator for continued culture for 12-14 hours.

After 12 hours, the cells were transferred to a 96-well plate, centrifuged at 800 g for 5 minutes, the supernatant was discarded, and then the cells were washed once with PBS and collected by centrifugation. 100 µL of Fixation and Permeabilization Solution (BD, 554722) was added to each well of the 96-well U-shape plate, and the plate was incubated in a 4 °C refrigerator for 30 min. After centrifugation at 800 g for 5 minutes, the cells were collected and washed twice with 1× Perm/Wash Buffer (BD, 554723). F488-labeled Z6 antibodies (Z6-AF488) were added at 100 µL per well, and the plate was incubated in a 4 °C refrigerator for 30 minutes. After centrifugation at 800 g for 3 minutes, the cells were collected and washed twice with 1× Perm/Wash Buffer. The cells were resuspended in 100 µL of PBS per well, and the proportion of virus-infected positive cells was detected using a flow cytometer.

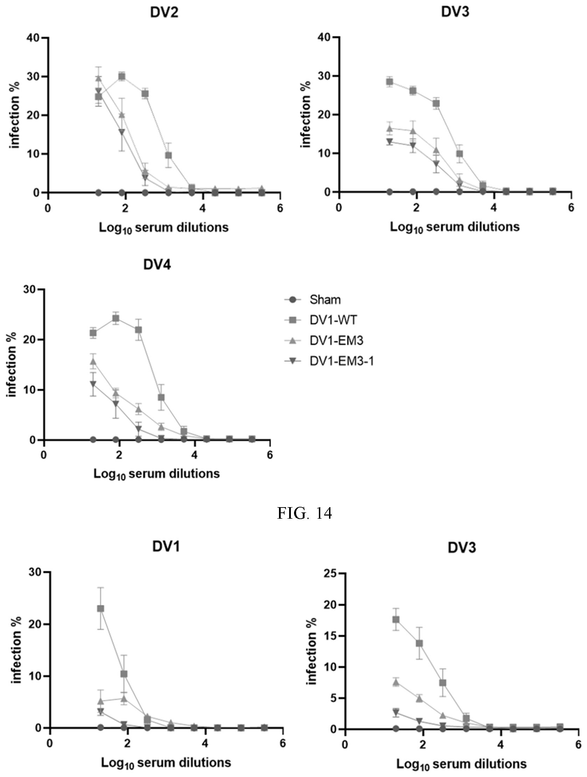

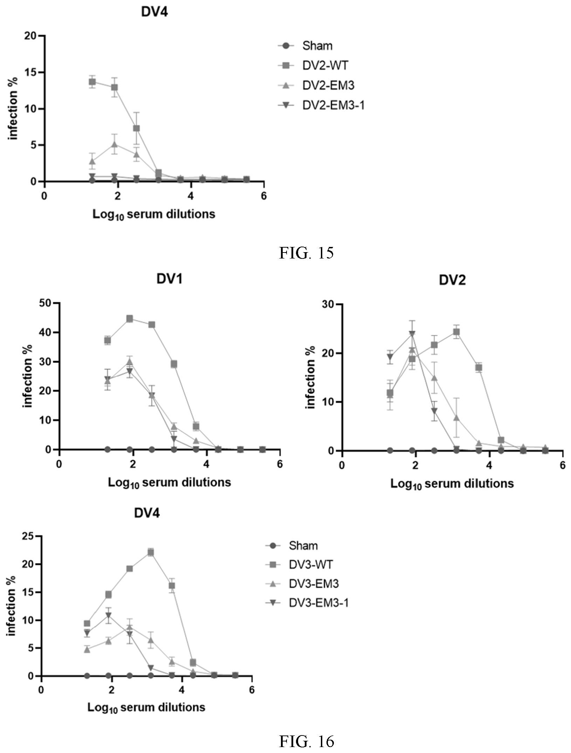

The results were shown in FIGS. 14-17, which showed that: DENV virus did not infect K562 cells when mixed with Sham immune sera. A strong ADE effect in vitro was detectable in the immune sera of the four wild-type vaccines (DV1-WT, DV2-WT, DV3-WT, and DV4-WT) at various dilutions, i.e., the WT vaccine sera enhanced the infection of K562 cells by Dengue virus, exhibiting an increased cell infection rate. Compared with the above wild-type vaccines, the ADE effect in vitro induced by the mouse sera of the mutant vaccines (DV1-EM3, DV2-EM3, DV3-EM3, DV4-EM3, DV1-EM3-1, DV2-EM3-1, DV3-EM3-1, and DV4-EM3-1) was significantly and substantially reduced, indicating that the risk of inducing the ADE effect by the mutant antigens was significantly and substantially reduced.

### Example 11: Evaluation of Vaccine-Induced ADE Effect In Vivo

In this example, the sera from BALB/c mice immunized with the vaccines DV4-WT, DV4-EM3, and DV4-EM3-1 obtained from Example 10 were mixed with DENV2 virus and then injected subcutaneously into AG129 mice to detect the ADE effect in vivo against DENV2 virus induced by the immune sera of the DENV4 vaccines in the AG129 mouse model.

The AG129 mice were used as the infection model, and the BALB/c mice were immunized with different vaccines or PBS. At 4 weeks after the booster immunization, blood was collected and the serum was separated, heat-inactivated, and the sera from different mice in each vaccine group were pooled, aliquoted, and stored at -80 °C until use. Before adoptive transfer to AG129 mice, the serum was first diluted 1000-fold to 50 µL with PBS, and then mixed with an equal volume of DENV2 virus (6000 FFU). The resulting virus-serum mixture was injected subcutaneously into AG129 mice. The survival rate and body weight loss of the mice were monitored daily, and mice that lost 25% of their initial body weight were euthanized.

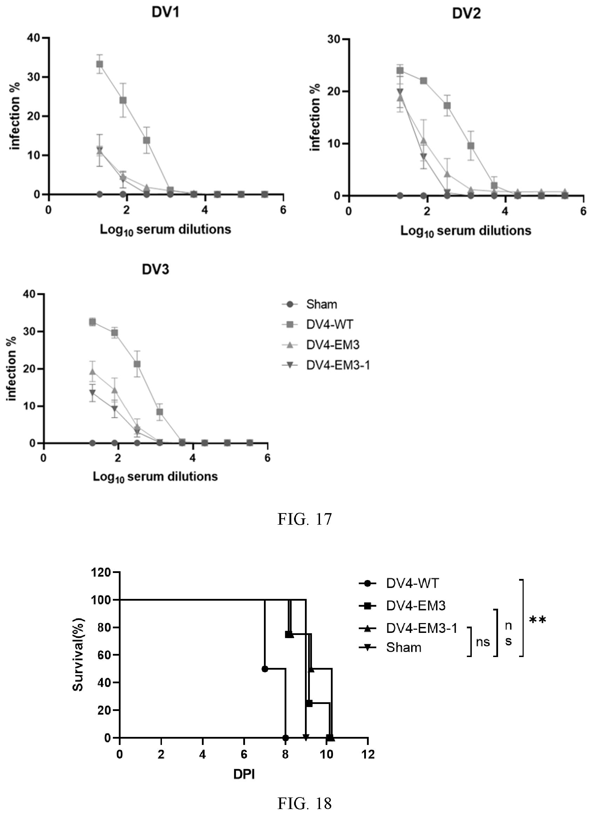

The results were shown in FIG. 18, which showed that compared with the adoptive transfer of PBS immune sera, the immune sera of the wild-type vaccine DV4-WT induced earlier death in AG129 mice, exhibiting a significant ADE effect. In contrast, the immune sera of the mutant vaccines DV4-EM3 and DV4-EM3-1 did not cause faster death in AG129 mice, showing no ADE effect.

### Example 12: Immunization of Mice with Tetravalent Dengue Virus mRNA Vaccines

In this example, female BALB/c mice aged 6-8 weeks (purchased from Beijing Vital River Laboratory Animal Technology Co., Ltd.) were used for the tetravalent Dengue virus vaccine immunization experiment. The experiment was divided into a negative control group (Sham group), a tetravalent wild-type vaccine group (tWT group), and a tetravalent mutant vaccine group (tMut group), with 8 mice per group, wherein, the negative control group was immunized with saline; the tWT vaccine was a mixture of four vaccines prepared in Example 1: DV1-WT, DV2-WT, DV3-WT, and DV4-WT, with each vaccine at 3 µg/dose, i.e., a total of 12 µg/dose; the tMut vaccine was a mixture of four vaccines prepared in Example 1: DV1-EM3-1, DV2-EM3-1, DV3-EM3, and DV4- EM3, with each vaccine at 3 µg/dose, i.e., a total of 12 µg/dose. Each immunization group was subjected to intramuscular injection on day 0 and day 21, respectively. Blood was collected at week 6 after the initial immunization, and the serum was separated at 4 °C, inactivated at 56 °C for 30 minutes, and stored at -80 °C for later use.

### Example 13: Expression and Purification of FL Antibody scFv

In this example, the Dengue virus FL antibody Z5-scFv (Wang, Q. H. et al. Sci Transl Med 8, (2016)) and Z6-scFv (Dai, L. et al. Nat Immunol 22, 958-968, (2021)) were respectively constructed according to the antibody heavy chain and light chain sequences disclosed in the literature. A signal peptide and a His tag were added at the 5' and 3' ends of the scFv antibody amino acid sequence, respectively, and then optimized according to mammalian cell codon bias to obtain the Z5-scFv and Z6-scFv antibody nucleic acid coding sequences as set forth in SEQ ID NO:125 and SEQ ID NO:126, respectively. Thereafter, a Kozak sequence and a stop codon were added at the 5' end and 3' end of the above nucleic acid coding sequences, respectively, and gene synthesis was commissioned to GenScript Biotech Corporation. The synthesized gene fragments were cloned into the pCAGGS vector by double digestion with 5'EcoRI and 3'XhoI to construct the E protein expression plasmids pCAGGS-Z5-scFv and pCAGGS-Z6-scFv. Protein expression was performed using HEK293F cells, and protein purification was performed using HisTrap^{™} excel affinity chromatography column and Superdex 200 Increase 10/300 GL gel filtration chromatography column.

HEK293F cells in healthy condition were selected, seeded into fresh SMM293-TII medium at a density of 1-1.5x10⁶ cells/mL one day prior to transfection, and cultured in a constant-temperature shaker at 37 °C, 5% CO₂, 150 rpm. On the day of transfection, the cell density was adjusted to 2.5×10⁶ cells/mL. To prepare the transfection reagent (using 10 mL of cells as an example), 10 µg of plasmid was diluted with 150 mM NaCl to a total volume of 250 µL and gently mixed. Sinofection transfection reagent was diluted with 150 mM NaCl to a total volume of 250 µL and gently mixed. The diluted plasmid and the transfection reagent were each allowed to stand separately for about 5 min, then gently mixed to a total volume of 500 µL, followed by standing at room temperature for another 10 min. The transfection mixture was added dropwise to the cell culture medium while gently shaking the culture flask. After mixing, the flask was returned to the shaker for continued culture. At 24 h after transfection, 350 µL of SMS-293-SUPI feed solution was added, and thereafter, the feed solution (350 µL) was added every 48 h. Five days after transfection, the supernatant was collected for protein purification.

The cell supernatant collected five days after transfection was centrifuged at 8000 rpm, 4 °C for 1 h, and then filtered through a 0.22 µm filter membrane. His affinity chromatography purification: the HisTrap^{™} excel affinity chromatography column was first rinsed with about 3-5 column volumes of water filtered through a 0.22 µm filter membrane. Thereafter, the column was rinsed with HisTrap affinity chromatography equilibration buffer until both the UV and conductivity detection baselines had stabilized, indicating that the column had been equilibrated. Then, the cell supernatant was passed through the column at a flow rate of 1-2 mL/min to allow the protein to bind to the column. After all the supernatant had passed through the column, the column was rinsed with 5-10 column volumes of affinity chromatography equilibration buffer until the UV detection baseline had stabilized. Finally, the column was rinsed sequentially with elution buffers containing 50 mM, 300 mM, and 1 M imidazole. The collected protein fractions were sampled and identified by SDS-PAGE. Further purification was performed using Superdex 200 Increase 10/300 GL gel filtration chromatography. The sample obtained from the affinity chromatography was concentrated to below 800 µL using a 10 kDa ultrafiltration concentrator and then transferred to a 1.5 mL centrifuge tube. After centrifugation at 12,000 g, 4 °C for 20 min, the sample was transferred to a new 1.5 mL centrifuge tube, ensuring that no precipitate was collected. Centrifugation was performed again at 12,000 g, 4 °C for 20 min to remove air bubbles from the sample, and the sample was ready for loading. The Superdex 200 Increase 10/300 GL gel filtration chromatography column was equilibrated with gel filtration chromatography equilibration buffer at a maximum flow rate of 0.5 mL/min until the UV detection baseline had stabilized, and the sample was loaded using a 1 mL loop. After loading, the column was rinsed with 20/50 equilibration buffer (20 mM Tris, 50 mM NaCl) at a flow rate of 0.5 mL/min. Peak collection was performed to collect the sample at the peak position. The collected sample was prepared and identified by SDS-PAGE. The sample identified to be correctly expressed was concentrated using a 10 kDa ultrafiltration concentrator, aliquoted, and stored in a -80°C freezer for later use.

### Example 14: Detection of FL Epitope Antibodies Induced by Tetravalent Dengue mRNA Vaccine by Competitive Enzyme-Linked Immunosorbent Assay (ELISA)

In this example, the sera of mice immunized with the tetravalent Dengue mRNA vaccine obtained from Example 12 were used to detect the antibody levels against the FL epitope of the antigen E protein induced by the tetravalent Dengue vaccine through competitive ELISA.

Specifically, the following procedure was applied:
(1) the DV E protein prepared in Example 3 was diluted to 2 µg/mL with ELISA coating buffer (Solarbio, C1050) and then added to a 96-well ELISA plate (Corning, 3590) with 100 µL per well; the plate was then placed at 4 °C for 12 hours;
(2) the coating buffer was discarded, and the plate was washed once with PBS; subsequently, 100 µL of a 5% skim milk solution prepared with PBS was added to each well of the 96-well plate as a blocking solution, and the plate was incubated at room temperature for 1 hour for blocking; after blocking, the plate was washed once with PBS;
(3) during the blocking in step (2), the scFv competing antibody mixture (Z5-scFv and Z6-scFv mixed at a concentration ratio of 1:1) was diluted to a concentration of 20 µg/mL with blocking solution; in the non-competitive ELISA, 50 µL of blank blocking solution was added; in the competitive ELISA, 50 µL of scFv competing antibody was added; blocking solution was added to the negative control; the plate was incubated at 37°C for 1 hour;
(4) during the blocking in step (3), the mouse immune serum samples were serially diluted 2-fold with blocking solution starting from a 10000-fold dilution; then 50 µL of the diluted immune serum was added to each well of the ELISA plate, with blocking solution added to the negative control; The plate was incubated at 37°C for 2 hours, and then washed 4 times with PBST;
(5) an HRP-conjugated goat anti-mouse secondary antibody (Abcam, ab6789) diluted 1:2000 with blocking solution was added and incubated at 37 °C for 1 hour, followed by washing 5-6 times with PBST; subsequently, TMB color development solution was added for color development; after an appropriate reaction time, 2 M hydrochloric acid was added to stop the reaction, and the OD₄₅₀ value was measured on a microplate reader.

The antibody titer was defined as the highest serum dilution at which the reaction value was greater than 2.1 times the negative control value. When the reaction value at the lowest dilution (detection limit) was still less than 2.1 times the background value, the titer of this sample was defined as half of the lowest dilution, i.e., 1:5.

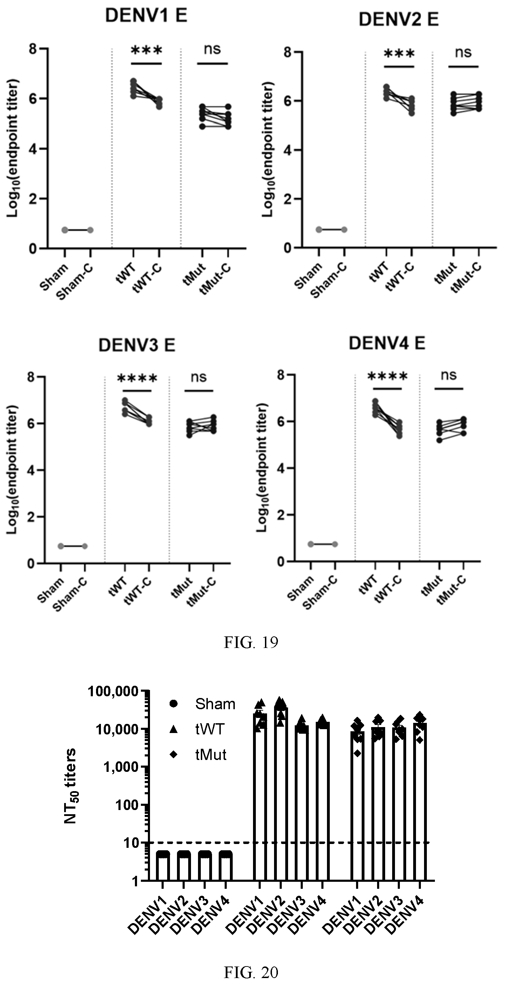

The competitive ELISA results were shown in FIG. 19, which showed: when the FL epitope scFv competing antibody was not added (tWT and tMut), all E protein-specific antibodies in the mouse sera could bind to the coated E protein; when the FL epitope scFv competing antibody was added (tWT-C and tMut-C), the FL epitope antibodies in the mouse sera failed to bind to the coated E protein, while antibodies against other epitopes could bind to the coated E protein normally; therefore, the difference between the two could represent the amount of FL epitope antibodies in the mouse sera; after adding the FL epitope scFv competing antibody, the antibody titers of the tWT vaccine sera were significantly and substantially reduced, indicating that the tWT vaccine induced the production of a large amount of FL epitope antibodies; in contrast, the antibody titers of the tMut vaccine sera measured with or without the addition of the FL epitope scFv competing antibody remained unchanged, indicating that the tMut vaccine did not induce FL epitope antibodies. The above results indicated that the mutant antigen of the present application did not induce FL antibodies with high ADE activity in mice, suggesting that it had a low ADE risk.

### Example 15: Detection of Neutralizing Antibodies Induced by the Tetravalent Dengue Vaccine

The neutralizing antibody titers against four serotypes of Dengue virus in the sera of mice immunized with the tetravalent Dengue mRNA vaccine obtained from Example 12 were quantitatively determined by Dengue virus micro-neutralization assay. The specific operation steps were as follows.

Vero cells were seeded in a 24-well cell culture plate one day in advance, and then used for the neutralization assay on the next day when the cells grew to a confluence of greater than 70%. The serum was serially diluted with DMEM medium (Invitrogen, C11995500BT) containing 1% FBS (Gibco, 10270-106), and the virus was also diluted with DMEM medium containing 1% FBS. The serum and the virus solution were mixed at an equal volume ratio (50 µL each). Meanwhile, the diluted virus was mixed with an equal volume of blank medium as a positive control. The mixtures were incubated at 37°C for 30 min. After incubation was complete, the supernatant medium in the Vero cell plate was removed, and the above mixture was added to the cells at 100 µL/well. The plate was placed in a 37°C incubator for continued culture for 3 days. After 3 days, the cell culture plate was taken out, all the supernatant was discarded, and the cells were washed once with PBS. The medium was removed, and the cells were washed once with PBS. 50 µL of fixation and permeabilization solution (BD Cytofix/Cytoperm Soln Kit 554714) was added to each well for fixation at 4°C for 30 min. The plate was washed twice with PBST, and 50 µL of MZ24-HRP dilution (a concentration of 2 µg/mL) was added to each well and incubated at 4°C for 1 h. The plate was washed three times with PBST, and 50 µL of TMB substrate solution was added to each well. After 8 min, the reaction was stopped with 2 M sulfuric acid, and the absorbance at 450 nm was measured using a microplate reader (PerkinElmer). Non-linear fitting of the data was performed using GraphPad Prism software to calculate the corresponding serum dilution factor when 50% neutralization of cell infection was achieved, i.e., the neutralizing titer (NT₅₀). When the serum at the lowest dilution still failed to neutralize 50% of cell infection, the NT₅₀ of the sample was defined as half of the lowest dilution.

The results were shown in FIG. 20, which showed that, compared with the negative control group, both the tWT and tMut vaccines induced high-titer neutralizing antibody immune responses against the four serotypes of Dengue virus. In particular, the tMut vaccine also induced a balanced neutralizing antibody response against the four serotypes of virus.

### Example 16: Evaluation of Challenge Protection of the Tetravalent Dengue Vaccine

In this example, a lethal AG129 mouse model was used to evaluate the challenge protection efficacy of the tetravalent Dengue mRNA vaccine.

Specifically, AG129 mice aged 6-8 weeks (purchased from Jiangsu GemPharmatech Co., Ltd.) were used for the tetravalent Dengue virus vaccine immunization and challenge experiment. The experiment was divided into a negative control group (Sham group) and a tetravalent mutant vaccine group (tMut group), with 12 mice per group; wherein, the negative control group was immunized with saline; the tMut vaccine was a mixture of four vaccines prepared in Example 1: DV1-EM3-1, DV2-EM3-1, DV3-EM3, and DV4-EM3, with each vaccine at 3 µg/dose, i.e., a total of 12 µg/dose. Each immunization group was subjected to intramuscular injection on day 0 and day 21, respectively. On day 49 after the initial immunization, the mice in each group were randomly divided into two equal halves, with one half being infected subcutaneously with 1×10⁶ FFU of DENV1 virus, and the other half being infected subcutaneously with 1×10⁶ FFU of DENV2 virus. After DENV virus challenge, the animals were weighed and monitored daily for clinical signs of disease. Mice that lost 25% of their initial body weight were euthanized for animal welfare reasons, and finally, the mortality rate was calculated.

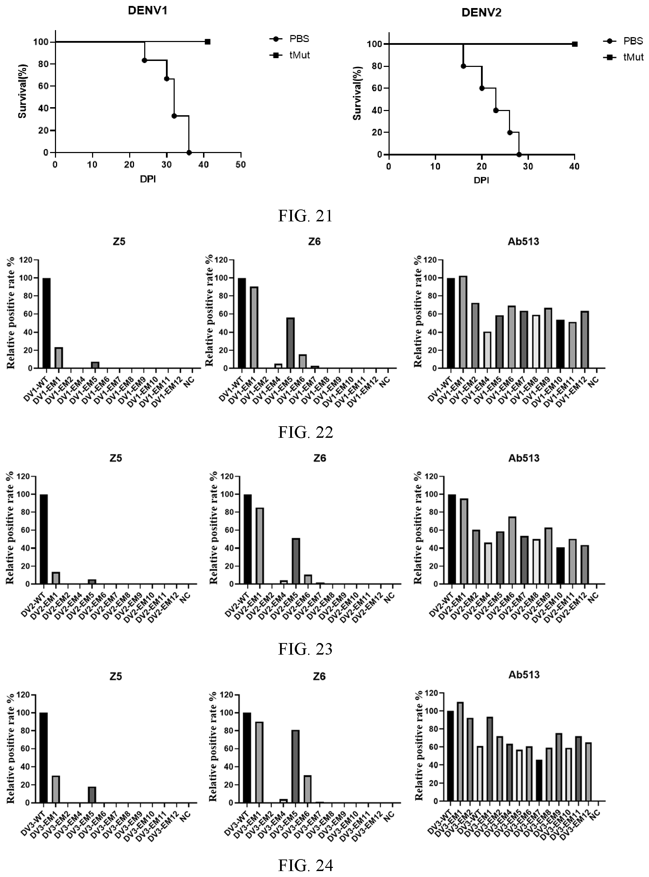

The results were shown in FIG. 21, which showed that after challenge with DENV1 and DENV2 viruses, all mice in the Sham group died, whereas all mice in the tMut vaccine group survived, indicating that the tMut vaccine could provide excellent immune protection for AG129 mice.

### Example 17: Preparation of Dengue Virus mRNA Vaccines

In this example, a series of mRNA constructs encoding the full-length Dengue virus prME protein (i.e., comprising prM protein + E protein) were designed, including:
DV1-WT (i.e., wild-type DV1), encoding the prME antigen protein as set forth in SEQ ID NO:1;
DV1-EM1, encoding the prME antigen protein as set forth in SEQ ID NO:2;
DV1-EM2, encoding the prME antigen protein as set forth in SEQ ID NO:5;
DV1-EM4, encoding the prME antigen protein as set forth in SEQ ID NO:6;
DV1-EM5, encoding the prME antigen protein as set forth in SEQ ID NO:7;
DV1-EM6, encoding the prME antigen protein as set forth in SEQ ID NO:8;
DV1-EM7, encoding the prME antigen protein as set forth in SEQ ID NO:9;
DV1-EM8, encoding the prME antigen protein as set forth in SEQ ID NO:10;
DV1-EM9, encoding the prME antigen protein as set forth in SEQ ID NO:11;
DV1-EM10, encoding the prME antigen protein as set forth in SEQ ID NO:12;
DV1-EM11, encoding the prME antigen protein as set forth in SEQ ID NO:13;
DV1-EM12, encoding the prME antigen protein as set forth in SEQ ID NO:14;
DV2-WT (i.e., wild-type DV2), encoding the prME antigen protein as set forth in SEQ ID NO:15;
DV2-EM1, encoding the prME antigen protein as set forth in SEQ ID NO:16;
DV2-EM2, encoding the prME antigen protein as set forth in SEQ ID NO:19;
DV2-EM4, encoding the prME antigen protein as set forth in SEQ ID NO:20;
DV2-EM5, encoding the prME antigen protein as set forth in SEQ ID NO:21;
DV2-EM6, encoding the prME antigen protein as set forth in SEQ ID NO:22;
DV2-EM7, encoding the prME antigen protein as set forth in SEQ ID NO:23;
DV2-EM8, encoding the prME antigen protein as set forth in SEQ ID NO:24;
DV2-EM9, encoding the prME antigen protein as set forth in SEQ ID NO:25;
DV2-EM10, encoding the prME antigen protein as set forth in SEQ ID NO:26;
DV2-EM11, encoding the prME antigen protein as set forth in SEQ ID NO:27;
DV2-EM12, encoding the prME antigen protein as set forth in SEQ ID NO:28;
DV3-WT (i.e., wild-type DV3), encoding the prME antigen protein as set forth in SEQ ID NO:29;
DV3-EM1, encoding the prME antigen protein as set forth in SEQ ID NO:30;
DV3-EM2, encoding the prME antigen protein as set forth in SEQ ID NO:33;
DV3-EM4, encoding the prME antigen protein as set forth in SEQ ID NO:34;
DV3-EM5, encoding the prME antigen protein as set forth in SEQ ID NO:35;
DV3-EM6, encoding the prME antigen protein as set forth in SEQ ID NO:36;
DV3-EM7, encoding the prME antigen protein as set forth in SEQ ID NO:37;
DV3-EM8, encoding the prME antigen protein as set forth in SEQ ID NO:38;
DV3-EM9, encoding the prME antigen protein as set forth in SEQ ID NO:39;
DV3-EM10, encoding the prME antigen protein as set forth in SEQ ID NO:40;
DV3-EM11, encoding the prME antigen protein as set forth in SEQ ID NO:41;
DV3-EM12, encoding the prME antigen protein as set forth in SEQ ID NO:42;
DV4-WT (i.e., wild-type DV4), encoding the prME antigen protein as set forth in SEQ ID NO:43;
DV4-EM1, encoding the prME antigen protein as set forth in SEQ ID NO:44;
DV4-EM2, encoding the prME antigen protein as set forth in SEQ ID NO:47;
DV4-EM4, encoding the prME antigen protein as set forth in SEQ ID NO:48;
DV4-EM5, encoding the prME antigen protein as set forth in SEQ ID NO:49;
DV4-EM6, encoding the prME antigen protein as set forth in SEQ ID NO:50;
DV4-EM7, encoding the prME antigen protein as set forth in SEQ ID NO:51;
DV4-EM8, encoding the prME antigen protein as set forth in SEQ ID NO:52;
DV4-EM9, encoding the prME antigen protein as set forth in SEQ ID NO:53;
DV4-EM10, encoding the prME antigen protein as set forth in SEQ ID NO:54;
DV4-EM11, encoding the prME antigen protein as set forth in SEQ ID NO:55;
DV4-EM12, encoding the prME antigen protein as set forth in SEQ ID NO:56.

Next, according to mammalian cell codon bias, the nucleic acid sequences encoding the above recombinant DV prME antigen proteins were optimized to obtain optimized nucleic acid coding sequences, wherein the optimized nucleic acid coding sequences of DV1-WT, DV2-WT, DV3-WT, and DV4-WT were as set forth in SEQ ID NOs:114-117, respectively; the optimized nucleic acid coding sequences of DV1-EM1, DV1-EM2, DV1-EM4, DV1-EM5, DV1-EM6, DV1-EM7, DV1-EM8, DV1-EM9, DV1-EM10, DV1-EM11, and DV1-EM12 were as set forth in SEQ ID NOs:62 and 65-74, respectively; the optimized nucleic acid coding sequences of DV2-EM1, DV2-EM2, DV2-EM4, DV2-EM5, DV2-EM6, DV2-EM7, DV2-EM8, DV2-EM9, DV2-EM10, DV2-EM11, and DV2-EM12 were as set forth in SEQ ID NOs:75 and 78-87, respectively; the optimized nucleic acid coding sequences of DV3-EM1, DV3-EM2, DV3-EM4, DV3-EM5, DV3-EM6, DV3-EM7, DV3-EM8, DV3-EM9, DV3-EM10, DV3-EM11, and DV3-EM12 were as set forth in SEQ ID NOs:88 and 91-100, respectively; the optimized nucleic acid coding sequences of DV4-EM1, DV4-EM2, DV4-EM4, DV4-EM5, DV4-EM6, DV4-EM7, DV4-EM8, DV4-EM9, DV4-EM10, DV4-EM11, and DV4-EM12 were as set forth in SEQ ID NOs:101 and 104-113, respectively. Then, a Kozak sequence and a signal peptide nucleic acid coding sequence (as set forth in SEQ ID NO:118, which encoded the signal peptide having an amino acid sequence as set forth in SEQ ID NO: 58) were added at the 5' end of each nucleic acid coding sequence, and a stop codon was added at the 3' end of each. Gene synthesis was commissioned to GenScript Biotech Corporation. The synthesized gene fragments were recombined into the pHRNT vector (the Invention Patent No. ZL202110224383.0) preserved in the laboratory to obtain template plasmids for preparing mRNA vaccines. The mRNA vaccine preparation method was performed with reference to Example 1.

### Example 18: Detection of Antigen Expression of Dengue Virus mRNA Vaccines

In this example, a series of Dengue virus prME mRNA vaccines prepared in Example 17 were transfected into 293T cells. Thereafter, the FL epitope antibody and other neutralizing epitope antibodies prepared in Example 2 were used to detect the status of each epitope and the protein expression level in each prME. Detailed experimental steps were performed with reference to Example 4. Cell fluorescence was detected on a BD FACSAria III flow cytometer, and the relative positive rate of each mutant vaccine was calculated with the positive cell rate of the WT vaccine as 100%.

The results were shown in FIGS. 22-25, which showed that:
for the DV1 mRNA vaccines, the FL epitope antibodies (Z5 and Z6) could bind to DV1-WT, but showed reduced binding or no binding to all DV1 mutants, indicating that the FL epitope had been partially or completely disrupted in all DV1 mutants; the neutralizing epitope antibody Ab513 could bind to both DV1-WT and all DV1 mutants, indicating that the neutralizing epitope was still present in all DV1 mutants, and all DV1 mutants had a high protein expression level.

Similar results were obtained for the DV2 mRNA vaccines, DV3 mRNA vaccines, and DV4 mRNA vaccines.

### Example 19: Evaluation of Challenge Protection of DV2 Mutant mRNA Vaccines

In this example, a lethal AG129 mouse model was used to evaluate the challenge protection efficacy of DV2 mRNA vaccines with different mutants prepared in Example 17 (DV2-EM1, DV2-EM2, DV2-EM4, DV2-EM5, DV2-EM6, DV2-EM7, DV2-EM8, DV2-EM9, DV2-EM10, DV2-EM11, and DV2-EM12).

Specifically, AG129 mice aged 6-8 weeks (purchased from Jiangsu GemPharmatech Co., Ltd.) were used for the vaccine immunization and challenge experiment. The experiment was divided into a negative control group (Sham group) and various mutant vaccine groups (DV2-EM1, DV2-EM2, DV2-EM4, DV2-EM5, DV2-EM6, DV2-EM7, DV2-EM8, DV2-EM9, DV2-EM10, DV2-EM11, and DV2-EM12 groups), with 5 mice per group. The negative control group was immunized with saline, and the mutant vaccines were administered at 15 µg/mouse/dose. Each immunization group was subjected to intramuscular injection on day 0 and day 21, respectively. On day 49 after the initial immunization, the mice were infected subcutaneously with 1×10⁶ FFU of DENV2 virus. After DENV virus challenge, the animals were weighed and monitored daily for clinical signs of disease. Mice that lost 25% of their initial body weight were euthanized for animal welfare reasons, and finally, the mortality rate was calculated.

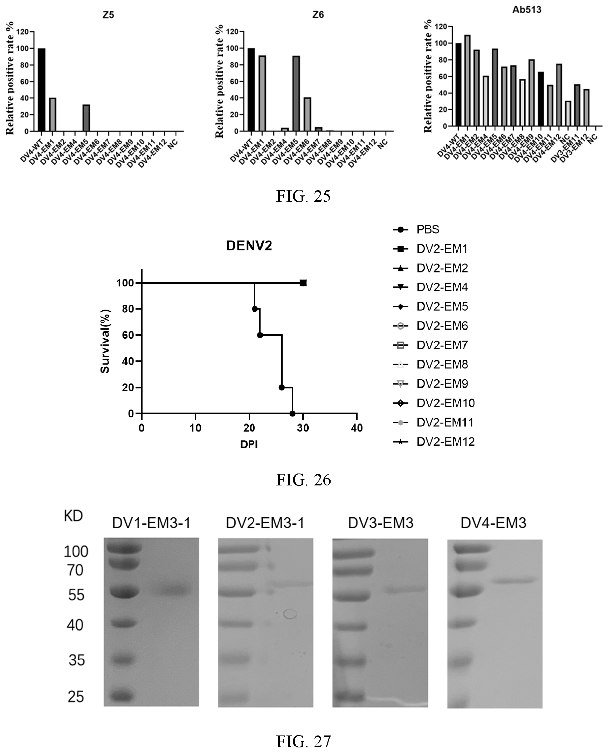

The results were shown in FIG. 26, which showed that after challenge with DENV2 virus, all mice in the Sham group died, whereas all mice in the DV2-EM1, DV2-EM2, DV2-EM4, DV2-EM5, DV2-EM6, DV2-EM7, DV2-EM8, DV2-EM9, DV2-EM10, DV2-EM11, and DV2-EM12 vaccine groups survived. These results indicated that each mutant vaccine could provide excellent immune protection for AG129 mice.

### Example 20: Preparation of Dengue Virus-Like Particle (VLP) Vaccines

For viruses belonging to the *Flavivirus* genus, recombinant expression of the full-length prME protein can form virus-like particles. In this example, a series of mammalian cell expression plasmids encoding the full-length prME protein of Dengue virus were designed, including:
DV1-EM3-1, encoding the prME antigen protein as set forth in SEQ ID NO:4;
DV2-EM3-1, encoding the prME antigen protein as set forth in SEQ ID NO:18;
DV3-EM3, encoding the prME antigen protein as set forth in SEQ ID NO:31;
DV4-EM3, encoding the prME antigen protein as set forth in SEQ ID NO:45;

Next, according to mammalian cell codon bias, the nucleic acid sequences encoding the above recombinant DV prME antigen proteins were optimized to obtain optimized nucleic acid coding sequences, wherein the optimized nucleic acid coding sequences of DV1-EM3-1, DV2-EM3-1, DV3-EM3, and DV4-EM3 were as set forth in SEQ ID NOs:64, 77, 89, and 102, respectively. Then, a Kozak sequence and a signal peptide nucleic acid coding sequence (as set forth in SEQ ID NO:118, encoding the signal peptide having an amino acid sequence as set forth in SEQ ID NO:58) were added at the 5' end of each nucleic acid coding sequence, and a stop codon was added at the 3' end. Gene synthesis was commissioned to GenScript Biotech Corporation. The synthesized gene fragments were recombined into the pCAGGS vector preserved in the laboratory to obtain template plasmids for preparing mRNA vaccines. HEK293T cells were used to express the virus-like particles. Purification of the virus-like particles was performed using a HiTrap Capto Q ImpRes anion exchange chromatography column and a Superose^{™} 6 Increase 10/300 GL gel filtration chromatography column. One day prior to transfection, 293T cells that had reached confluence in a 15 cm culture dish were passaged at a ratio of 1:3. After 14-16 h, when the cell density reached more than 70%, transfection was performed. Preparation of the transfection system: For each 15 cm culture dish, 30 µg of plasmid was added to 500 µL of opti-MEM, gently mixed, and allowed to stand for 5 min. 150 µL of Sinofection^{®} Transfection Reagent was added to 350 µL of opti-MEM, gently mixed, and allowed to stand for 5 min. Then, the two were mixed together and incubated at room temperature for 15 min. The transfection system was mixed and slowly added dropwise to the antibiotic-free cell culture supernatant in the 15 cm culture dish, gently mixed, and placed in a 37 °C, 5% CO₂ incubator for culture. After 4-6 h, the cell culture supernatant containing the transfection reagent was discarded and replaced with an antibiotic-free, serum-free DMEM medium. The culture was continued for 48 h, and then the cells and expression supernatant were harvested.

A concentration cup was prepared, and a filter membrane with a molecular weight cutoff of 100 kDa was selected according to the size of VLP. The cell supernatant collected at 2 days after transfection was centrifuged at 3500 rpm, 4 °C for 15 min. The supernatant was then poured into the concentration cup, and the liquid nitrogen valve was slowly opened to concentrate the supernatant to 5-10 mL. The solution was exchanged to 20 mM Tris/150 mM NaCl containing 0.1 mM EDTA at pH 8.0.

HiTrap Capto Q ImpRes anion exchange chromatography purification: A HiTrap Capto Q ImpRes anion exchange chromatography column was first rinsed with about 3-5 column volumes of water filtered through a 0.22 µm filter membrane. Thereafter, the column was rinsed with 20 mM Tris/150 mM NaCl equilibration buffer until both the UV and conductivity detection baselines had stabilized, indicating that the column had been equilibrated. Then, the concentrated and buffer-exchanged cell supernatant was passed through the column at a flow rate of 1 mL/min to allow impurity proteins to bind to the column, and the flow-through was collected. After all the supernatant had passed through the column, the column was rinsed with 5-10 column volumes of affinity chromatography equilibration buffer until the UV detection baseline had stabilized. Finally, the column was rinsed sequentially with elution buffers containing 300 mM, 500 mM, and 1 M NaCl. The samples collected from the flow-through were prepared and identified by SDS-PAGE. Further purification was performed using a Superose^{™} 6 Increase 10/300 GL gel filtration chromatography column. The sample obtained from anion exchange chromatography was concentrated to below 1000 µL using a 100 kDa ultrafiltration concentrator and then transferred to a 1.5 mL centrifuge tube. After centrifugation at 12,000 rpm, 4 °C for 20 min, the sample was transferred to a new 1.5 mL centrifuge tube, ensuring that no precipitate was collected. Centrifugation was performed again at 12,000 rpm, 4 °C for 20 min to remove air bubbles in the sample, and the sample was ready for loading. The Superose^{™} 6 Increase 10/300 GL gel filtration chromatography column was equilibrated with 20 mM Tris/150 mM NaCl at a maximum flow rate of 0.5 mL/min until the UV detection baseline had stabilized, and the sample was loaded using a 1 mL loop. After loading, the column was rinsed with 20 mM Tris/150 mM NaCl equilibration buffer at a flow rate of 0.5 mL/min. Peak collection was performed to collect the sample at the peak position. The collected sample was prepared and preliminarily identified by SDS-PAGE:
1) 20 µL of the purified sample was taken, 5 µL of 5× protein loading buffer was added, and the mixture was heated at 100 °C for 3 min;
2) 10 µL of the supernatant was subjected to SDS-PAGE electrophoresis;
3) Coomassie Brilliant Blue staining was performed;
4) destaining with destaining solution was observed.

The identification results were shown in FIG. 27, which showed that the four virus-like particle vaccines DV1-EM3-1, DV2-EM3-1, DV3-EM3, and DV4-EM3 exhibited a single band only between 70 and 55 kDa in SDS-PAGE gel electrophoresis, which was consistent with expectations, indicating high sample purity.

### Example 21: Characterization of Dengue Virus-Like Particle Vaccines Using Transmission Electron Microscopy

In this example, the morphology of the Dengue virus-like particles prepared in Example 20 was observed and detected by transmission electron microscopy. The specific operation steps were as follows.

A copper grid, a phosphotungstic acid staining solution, a filter paper, and a sample box were prepared.
1) Preparation of negatively stained samples: first, glow discharge of the copper grid was performed. A glow discharge apparatus was commonly used for hydrophilic treatment of carbon-coated copper grids.
2) After glow discharge of the copper grid, the grid was picked up with sharp tweezers fixed with a long-tail clip.
3) 6.5 µL of sample was dropped onto the copper grid, allowed to stand for one minute, and then blotted away with the filter paper.
4) 6.5 µL of phosphotungstic acid was dropped onto the copper grid and immediately blotted away with the filter paper.
5) 6.5 µL of phosphotungstic acid was dropped onto the copper grid, allowed to stand for one minute, and the excess staining solution was blotted away with a wet filter paper.
6) After drying, electron microscopy observation was performed.

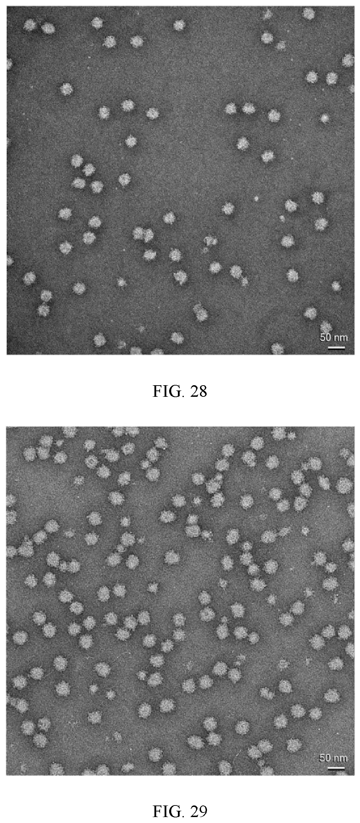

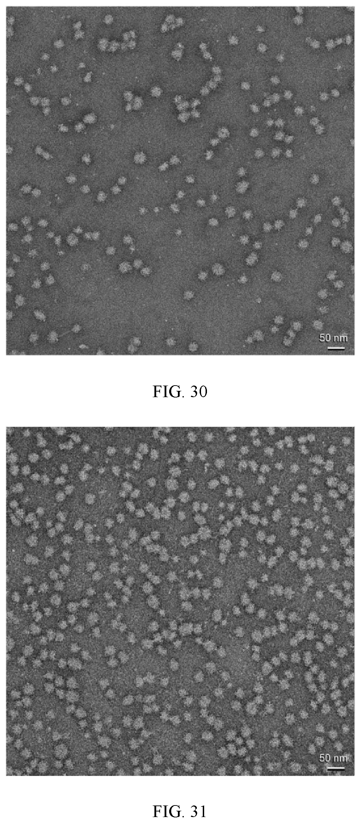

After negative staining with phosphotungstic acid was performed on the samples purified by chromatography, the samples were observed and analyzed by 100 kV electron microscopy. The electron microscopy results were shown in FIGS. 28-31, which showed that the negative staining results of the DV1-EM3-1, DV2-EM3-1, DV3-EM3, and DV4-EM3 virus-like particles indicated that a large number of uniform virus-like particles with a diameter of about 30 nm could be observed in the field of view.

### Example 22: Detection of Immunogenicity of Monovalent Dengue Virus Recombinant Protein Vaccines

Female BALB/c mice aged 6-8 weeks (purchased from Beijing Vital River Laboratory Animal Technology Co., Ltd.) were used for the vaccine immunization experiment. The experiment was divided into a monovalent Dengue virus recombinant protein vaccine immunization group and a negative control group (saline, as the Sham group), with 5 mice per group. For each immunization group, a placebo or the Dengue virus-like particle protein prepared in Example 20 mixed with a combined adjuvant consisting of Al(OH)₃ (Invivogen, vac-alu-250) and CpG 1826 (Invivogen, vac-1826-1) was administered by intramuscular injection on day 0 and day 21 at a dose of 12 µg/dose. Blood was collected at week 2 after the booster immunization, and the serum was separated at 4 °C, inactivated at 56 °C for 30 minutes, and stored at -80°C for later use. The method for detecting serum neutralizing antibody titers was performed with reference to Example 7.

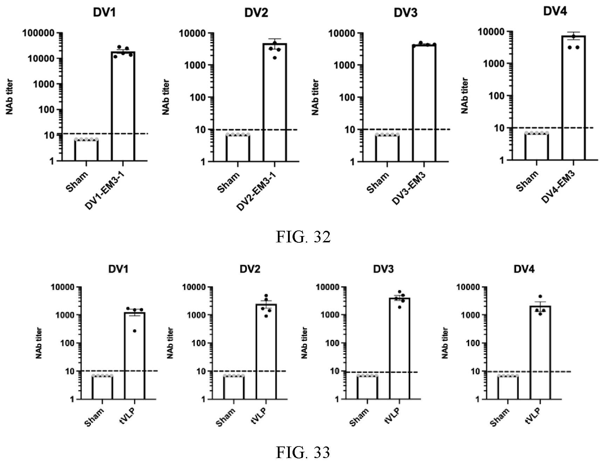

The neutralizing antibody titer detection results were shown in FIG. 32, which showed that no neutralizing antibodies were detected in the sera of the Sham group, whereas all mutant Dengue virus recombinant protein vaccines showed high neutralizing antibody titers against their respective viruses ranging from 4,000 to 20,000, indicating that the above Dengue virus recombinant protein vaccines had good immunogenicity.

### Example 23: Detection of Immunogenicity of Tetravalent Dengue Virus Recombinant Protein Vaccine

Female BALB/c mice aged 6-8 weeks (purchased from Beijing Vital River Laboratory Animal Technology Co., Ltd.) were used for the vaccine immunization experiment. The experiment was divided into a tetravalent Dengue virus recombinant protein vaccine group (tVLP) and a negative control group (i.e., Sham group), with 5 mice per group. The tVLP vaccine preparation method was as follows: the four virus-like particles DV1-EM3-1, DV2-EM3-1, DV3-EM3, and DV4-EM3 prepared in Example 20 were mixed in equal proportions at a mass ratio of 1:1:1:1, with each at 2.5 µg/dose, i.e., a total of 10 µg/dose, and then mixed with a combined adjuvant consisting of Al(OH)₃ (Invivogen, vac-alu-250) and CpG 1826 (Invivogen, vac-1826-1). A placebo or the vaccine was administered by intramuscular injection on day 0 and day 21, respectively. Blood was collected at 2 weeks after the booster immunization, and the serum was separated by centrifugation, inactivated by heating at 56 °C for 30 minutes, and stored at -80 °C for later use. The method for detecting serum neutralizing antibodies was performed with reference to Example 7.

The neutralization test results were shown in FIG. 33, which showed that no neutralizing antibodies against the four serotypes of Dengue virus were detected in the sera of the Sham group, whereas the tVLP vaccine induced a balanced and strong neutralizing antibody immune response against the four serotypes of Dengue virus, with neutralizing antibody titers ranging from 1,100 to 4,000, indicating that the tetravalent Dengue virus recombinant protein vaccine composed of mutant virus-like particles had good immunogenicity.

Finally, it should be noted that the above examples are only used to illustrate the technical solutions of the present application and not to limit it; although the present application has been described in detail with reference to the foregoing examples, it will be understood by those skilled in the art that the technical solutions described in the foregoing examples can still be modified or some technical features can be equivalently substituted; however, these modifications or substitutions do not make the essence of the corresponding technical solutions depart from the spirit and scope of the present application.

### Industrial Applicability

The recombinant antigen against Zika/Dengue virus provided in the present application can avoid inducing the production of FL epitope antibodies, thereby achieving the purpose of reducing or eliminating the ADE effect. Moreover, the recombinant antigen shows a correct E protein conformation, exhibits a protein expression level similar to or higher than that of the wild-type, and can induce high specific binding antibody titers and neutralizing antibody titers in animals, thereby providing effective immune protection, good clinical application value and industrialization prospects.

## Claims

1. A recombinant antigen having a full-length E protein sequence of Zika virus or Dengue virus, wherein the E protein has site mutations selected from the group consisting of:
a G106 site mutation in the FL fusion loop region;
W101 and G106 double site mutations in the FL fusion loop region;
W101 and G106 double site mutations in the FL fusion loop region and a mutation at amino acid position 125 in the non-FL fusion loop region;
G102 and G106 double site mutations in the FL fusion loop region;
N103 and G106 double site mutations in the FL fusion loop region;
W101, N103 and G106 triple site mutations in the FL fusion loop region.

2. The recombinant antigen according to claim 1, wherein the G106 site mutation in the FL fusion loop region is: G106V mutation;
and/or, the W101 and G106 double site mutations in the FL fusion loop region are selected from the group consisting of: (1) W101R and G106V, (2) W101G and G106V, and (3) W101L and G106V;
and/or, the W101 and G106 double site mutations in the FL fusion loop region and the mutation at amino acid position 125 in the non-FL fusion loop region are: W101R and G106V double site mutations in the FL fusion loop region and a mutation of the amino acid at position 125 in the non-FL fusion loop region to valine;
and/or, the G102 and G106 double site mutations in the FL fusion loop region are: G102R and G106V double site mutations;
and/or, the N103 and G106 double site mutations in the FL fusion loop region are selected from the group consisting of: (1) N103T and G106V, (2) N103H and G106V, (3) N103K and G106V, (4) N103P and G106V, and (5) N103Y and G106V;
and/or, the W101, N103 and G106 triple site mutations in the FL fusion loop region are selected from the group consisting of: (1) W101G, N103T and G106V, and (2) W101G, N103K and G106V.

3. The recombinant antigen according to claim 2, wherein the recombinant antigen has a full-length E protein sequence of Dengue virus serotype 1, and the E protein has site mutations selected from the group consisting of:
a G106V mutation;
W101R and G106V double site mutations;
W101G and G106V double site mutations;
W101L and G106V double site mutations;
W101R, G106V and L125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

4. The recombinant antigen according to claim 2, wherein the recombinant antigen has a full-length E protein sequence of Dengue virus serotype 2, and the E protein has a site mutation selected from the group consisting of:
a G106V mutation;
W101R and G106V double site mutations;
W101G and G106V double site mutations;
W101L and G106V double site mutations;
W101R, G106V and M125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

5. The recombinant antigen according to claim 2, wherein the recombinant antigen has a full-length E protein sequence of Dengue virus serotype 3, and the E protein has a site mutation selected from the group consisting of:
a G106V mutation;
W101R and G106V double site mutations;
W101G and G106V double site mutations;
W101L and G106V double site mutations;
W101R, G106V and I125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

6. The recombinant antigen according to claim 2, wherein the recombinant antigen has a full-length E protein sequence of Dengue virus serotype 4, and the E protein has a site mutation selected from the group consisting of:
a G106V mutation;
W101R and G106V double site mutations;
W101G and G106V double site mutations;
W101L and G106V double site mutations;
W101R, G106V and I125V triple site mutations;
G102R and G106V double site mutations;
N103T and G106V double site mutations;
N103H and G106V double site mutations;
N103K and G106V double site mutations;
N103P and G106V double site mutations;
N103Y and G106V double site mutations;
W101G, N103T and G106V triple site mutations;
W101G, N103K and G106V triple site mutations.

7. The recombinant antigen according to any one of claims 1 to 6, wherein when the recombinant antigen has the full-length E protein sequence of Zika virus, the recombinant antigen further comprises a full-length or partial prM protein sequence or M protein sequence of Zika virus.

8. The recombinant antigen according to any one of claims 1 to 6, wherein when the recombinant antigen has the full-length E protein sequence of Dengue virus, the recombinant antigen further comprises a full-length or partial prM protein sequence or M protein sequence of the corresponding serotype of Dengue virus;
preferably, when the recombinant antigen has the full-length E protein sequence of Dengue virus serotype 1, the recombinant antigen has the amino acid sequence as set forth in SEQ ID NOs: 2-14; and/or, when the recombinant antigen has the full-length E protein sequence of Dengue virus serotype 2, the recombinant antigen has the amino acid sequence as set forth in SEQ ID NOs: 16-28; and/or, when the recombinant antigen has the full-length E protein sequence of Dengue virus serotype 3, the recombinant antigen has the amino acid sequence as set forth in SEQ ID NOs: 30-42; and/or, when the recombinant antigen has the full-length E protein sequence of Dengue virus serotype 4, the recombinant antigen has the amino acid sequence as set forth in SEQ ID NOs: 44-56.

9. A method for preparing the recombinant antigen according to any one of claims 1 to 8, comprising the following steps:
adding a Kozak sequence and a signal peptide coding sequence at the 5' end, and a histidine tag coding sequence and a stop codon at the 3' end of the nucleotide sequence encoding the recombinant antigen of any one of claims 1-8; performing cloning and expression; screening for correct recombinants; transfecting the recombinants into expression system cells for expression; collecting the cell culture supernatant; and isolating the recombinant antigen from the cell culture supernatant.

10. The preparation method according to claim 9, wherein the expression system cell is a mammalian cell, an insect cell, a yeast cell, or a bacterial cell;
optionally, the mammalian cell is an HEK293T cell, a 293F series cell, or a CHO cell; further
optionally, the 293F series cell is an HEK293F cell, a Freestyle293F cell, or an Expi293F cell;
optionally, the insect cell is an sf9 cell, an Hi5 cell, an sf21 cell, or an S2 cell;
optionally, the yeast cell is a *Pichia pastoris* cell or a yeast cell modified therefrom;
optionally, the bacterial cell is an *Escherichia coli* cell.

11. A polynucleotide encoding the recombinant antigen according to any one of claims 1 to 8.

12. The polynucleotide according to claim 11, wherein the polynucleotide is DNA or mRNA, preferably mRNA;
preferably, the polynucleotide is a DNA having a sequence as set forth in any one of SEQ ID NOs: 62-113, or an mRNA corresponding to the DNA.

13. An expression cassette, a recombinant vector, a transgenic cell line, a recombinant bacterium, or a recombinant virus comprising the polynucleotide according to claim 11 or 12.

14. A vaccine composition, comprising the recombinant antigen according to any one of claims 1 to 8, or the polynucleotide according to claim 11 or 12, or the expression cassette, the recombinant vector, the transgenic cell line, the recombinant bacterium, or the recombinant virus according to claim 13, as an active ingredient.

15. The vaccine composition according to claim 14, wherein the vaccine composition is a recombinant protein vaccine, comprising the recombinant antigen according to any one of claims 1 to 8 and an adjuvant;
preferably, the adjuvant is one or more selected from the group consisting of: an aluminum adjuvant, an MF59 adjuvant, an MF59-like adjuvant, a CpG adjuvant, a QS-21-containing adjuvant, AS series adjuvants, and nanoparticle adjuvants.

16. The vaccine composition according to claim 15, wherein the vaccine composition is a DNA vaccine, comprising:
(i) a eukaryotic expression vector; and
(ii) a DNA sequence encoding the recombinant antigen according to any one of claims 1 to 8, constructed into the eukaryotic expression vector;
preferably, the DNA sequence encoding the recombinant antigen according to any one of claims 1 to 8 is the DNA sequence as set forth in any one of SEQ ID NOs: 62-113;
and/or, the eukaryotic expression vector is selected from the group consisting of pGX0001, pVAX1, pCAGGS, and pcDNA series vectors.

17. The vaccine composition according to claim 14, wherein the vaccine composition is an mRNA vaccine, comprising:
(I) an mRNA sequence encoding the recombinant antigen according to any one of claims 1 to 8; and
(II) a lipid nanoparticle;
preferably, the mRNA sequence encoding the recombinant antigen according to any one of claims 1 to 8 is an mRNA sequence corresponding to the DNA sequence as set forth in any one of SEQ ID NOs: 62-113.

18. The vaccine composition according to claim 14, wherein the vaccine composition is a viral vector vaccine, comprising:
(1) a viral backbone vector; and
(2) a DNA sequence encoding the recombinant antigen according to any one of claims 1 to 8, constructed into the viral backbone vector;
preferably, the DNA sequence encoding the recombinant antigen according to any one of claims 1 to 8 is the DNA sequence as set forth in any one of SEQ ID NOs: 62-113;
and/or, the viral backbone vector is selected from one or more of the following viral vectors: an adenoviral vector, a lentiviral vector, a poxvirus vector, an influenza virus vector, and an adeno-associated viral vector.

19. The vaccine composition according to any one of claims 14 to 18, wherein the vaccine composition is in the form of a nasal spray, an oral formulation, a suppository, or a parenteral formulation;
preferably, the nasal spray is selected from the group consisting of an aerosol, a spray, and a dry powder inhaler;
preferably, the oral formulation is selected from the group consisting of a tablet, a powder, a pill, a granule, a soft/hard capsule, a film coating, and an ointment;
further preferably, the tablet is a sublingual tablet;
further preferably, the granule is a fine granule;
further preferably, the powder is a pulvis;
further preferably, the pill is a pilule;
preferably, the parenteral formulation is a transdermal formulation, an ointment, a plaster, a topical liquid, an injectable formulation; further preferably, the injectable formulation is a bolus injectable formulation.

20. Use of the recombinant antigen according to any one of claims 1 to 8, the polynucleotide according to claim 11 or 12, or the expression cassette, the recombinant vector, the transgenic cell line, the recombinant bacterium, or the recombinant virus according to claim 13 in the manufacture of a medicament for detecting, preventing and/or treating Zika virus or Dengue virus infection;
preferably, the medicament is a vaccine.
